(19) **Europäisches Patentamt**
**European Patent Office**
**Office européen des brevets**

(11) **EP 4 704 088 A1**

(12) **EUROPEAN PATENT APPLICATION**

(43) Date of publication:
04.03.2026 Bulletin 2026/10

(51) International Patent Classification (IPC):
**G16B 40/10** (2019.01)        **G16B 40/20** (2019.01)

(21) Application number: 24197377.5

(22) Date of filing: 29.08.2024

(52) Cooperative Patent Classification (CPC):
**G16B 40/10; G01N 33/6848; G16B 40/20; H01J 49/0036**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: MSAID GmbH
**85748 Garching b. München (DE)**

(72) Inventors:
• **GESSULAT, Siegfried Friedel**
**85748 Garching b. München (DE)**
• **MAMISASHVILI, Lizi**
**85748 Garching b. München (DE)**

(74) Representative: **Boult Wade Tennant LLP**
**Salisbury Square House**
**8 Salisbury Square**
**London EC4Y 8AP (GB)**

(54) **METHOD AND APPARATUS FOR IDENTIFYING ONE OR MORE ANALYTES REPRESENTED IN CHIMERIC MASS SPECTROMETRY DATA**

(57) A method of identifying one or more analytes represented in mass spectrometry data, the method comprising: obtaining a set of candidate molecular sequences for the mass spectrometry data, wherein each candidate molecular sequence corresponds to a respective candidate analyte; transforming the mass spectrometry data into a mass spectrometry data vector in a latent space; transforming each candidate molecular sequence into a respective candidate molecular sequence vector in the latent space; determining a set of analyte coefficients for the set of candidate molecular sequences, based on the mass spectrometry data vector and the candidate molecular sequence vectors; providing, for one or more of the candidate molecular sequences, a respective indication of a match in the mass spectrometry data, based at least in part on the set of analyte coefficients.

Figure 2

**Description**

**Field of the invention**

The present invention relates to identification of analytes present in mass spectrometry data. In particular, methods and systems for identifying one or more analytes represented in (chimeric) mass spectrometry data and methods and systems for training a machine learning system to identify one or more analytes represented in (chimeric) mass spectrometry data.

**Background of the invention**

Mass spectrometry (MS) is an analytical technique that is widely used in industry. It's many industrial applications include identifying unknown compounds, quantifying known compounds, and determining structure and/or chemical properties of molecules.

Such analysis via mass spectrometry is performed using a mass spectrometer, which typically comprises three parts: an ion source; a mass analyzer; and a detector. The ion source is used to convert a portion of a sample (or 'precursor') to be analysed into ions. The mass analyser is then used to separate the ions according to their mass-to-charge (m/z) ratio. The detector detects the separated ions, and thereby collects data for calculating the abundances of each ion present at each m/z ratio. In this way, the mass spectrometer can be used to generate a mass spectrum.

A mass spectrum is a list of m/z ratios and their corresponding intensity (or relative abundance) values. A pair of m/z ratio and intensity value is termed a peak. A mass spectrum will have peaks at certain m/z ratios, indicating a greater abundance of ions at that m/z ratio. Patterns of peaks provide a signature which may allow an (unknown) compound (or compounds) in the sample to be identified.

For mass spectra containing a single analyte, this identification may involve looking up known mass spectrum signatures of candidate (or potential) analytes, for example in a library or a database, and comparing the mass spectrum signatures of known analytes to the measured (unknown) mass spectrum - where there is a match, the unknown analyte can be identified as the known analyte with the matching mass spectrum signature.

For mass spectra containing more than one analyte (chimeric mass spectra), the measured mass spectrum signature is a combination of the mass spectrum signatures of the constituent analytes. If the constituent analytes are known, their relative amounts can be determined by identifying a relative scaling between the mass spectrum peaks of the known constituent analytes to the mass spectrum peaks of the measured spectrum.

However, for some applications, both the constituent analytes of a mass spectrum containing more than one analyte and their relative amounts are unknown. For these applications, known methods may not be sufficient since a particular measured mass spectrum signature may have many possible matches for different analyte combinations in different relative amounts.

One such application may be bottom-up proteomics - namely, the characterisation of proteins by analysis of peptides released from the proteins through proteolysis. More specifically, bottom-up proteomics typically involves extracting proteins (e.g. from tissue) which are then proteolytically digested using a peptidase (e.g. with a specific cleavage pattern) to obtain peptides (i.e. shorter poly-amino-acid chains). After digestion, the digested mixture (including the produced peptides) may undergo further processes such as: sample clean-up in order to remove unwanted salts or other reagents from the sample; offline fractionation (e.g. liquid chromatography) to decrease sample complexity; enrichment of specific classes of (modified) peptides using physicochemical properties or antibodies; and/or labelling using isobaric labelling reagents (e.g. tandem mass tags TMT, TMTpro, iTRAQ).

Typically, in bottom-up proteomics a sample of a digested mixture is ionized and introduced into a first mass spectrometry instrument (MS1) which separates (or sorts) the peptide ions by m/z value. A certain ion population (i.e. the ions having an m/z value within a particular range) are then isolated from the output from the MS1 instrument and input into a collision cell. In the collision cell, the peptide ions collide with inert gas to cause fragmentation of the peptides (e.g. physical breakage of the peptides at the peptide bonds). The resulting peptide fragments tend to differ by virtue of the number of amino acids present. The resulting peptide fragments are then input into a second mass spectrometry instrument (MS2) which can be used to separate the different fragments by m/z value. A detector of the second mass spectrometry instrument (MS2) can then measure the relative abundances of the different fragments at the different m/z values to produce a mass spectrum. It will be appreciated that, often, the first mass spectrometry instrument (MS1) and the second mass spectrometry instrument (MS2) are performed by the same mass spectrometer hardware operating in a first and second mode respectively. The use of an MS1 instrument/operation followed by an MS2 instrument/operation is described as 'tandem mass spectrometry' (or 'MS/MS mass spectrometry').

In some applications it is desired to analyse the peptide fragments of a single peptide. However, this would require isolating at a single m/z value, or a very narrow m/z range of the MS1 instrument output for further analysis by MS2 which is often not feasible/possible. Instead, in practice, typically more than one peptide is input into the collision cell (and results in fragments for MS2 analysis), with the number of different peptides input being dependent on the m/z value range isolated. Accordingly, in practice, multiple peptides are co-isolated and co-fragmented during data acquisition. The resulting mass spectrum therefore has peaks which originate from more than one peptide and is said to be 'chimeric' (i.e. a spectrum from more than one isolated precursor). The relative amounts of the different peptides analysed by MS2 are generally unknown and therefore such bottom-up proteomics poses the problem of identifying the constituent peptides of a mass spectrum containing more than one peptide when their relative amounts are unknown. This problem can also be framed as identifying one or more (unknown) peptides represented in chimeric mass spectrometry data.

There are a number of existing data analysis methods which aim to solve this problem and which are independent of the instrument settings.

In the 'duplication' approach, the MS2 spectrum is submitted multiple times to the scoring algorithm, each time with a different peptide candidate mass, and hence candidate space. For every duplicated spectrum, the highest scoring match (e.g. the peptide candidate whose mass spectrum is most similar to a mass spectrum under analysis, based on heuristic criteria) is identified. Hence, this 'duplication' solution is prone to error since peptide fragments at a given m/z value (represented by a given peak) may be attributed to more than one candidate peptide.

Typically, 'matched' peptides are only identified as present in the sample under analysis if they 'score' above a particular threshold as described at Sinitcyn, P. et al, Computational Methods for Understanding Mass Spectrometry-Based Shotgun Proteomics Data. Annu. Rev. Biomed. Data Sci., 2018. 1(1): p. 207-234 which is incorporated herein in its entirety (and which can be accessed from https://www.annualreviews.org/doi/10.1146/annurev-biodatas ci-080917-013516).

In the 'subtraction' approach, the highest scoring match (e.g. the peptide candidate whose mass spectrum is most similar to a mass spectrum under analysis, based on heuristic criteria) is identified as the first isolated peptide. Then, the peaks matching the first identified isolated peptide are removed from the mass spectrometry data. The remaining mass spectrometry data is then analysed for 'matches' again to identify a second isolated peptide in the same way, and the process is repeated as required. Again, typically peptides are only identified if they 'score' above a particular threshold, and this limits the number of iterations required. However, the 'subtraction' solution is also prone to error since, for example, peaks sharing the same m/z ratio might be attributed to a first 'match' and not attributed to a second 'match'. This 'subtraction' solution is described in Dorfer V. et al, CharmeRT: Boosting Peptide Identifications by Chimeric Spectra Identification and Retention Time Prediction, J. Proteome Res. 2018, 17, 8, 2581-2589, which is incorporated herein by reference in its entirety (and which can be accessed from https://doi.org/10.1021/acs.jproteome.7b00836).

In other approaches, deconvolution of mass spectrometry data has been used for identification.

It will be appreciated that while the above approaches have been described in connection with proteomics, all of the above approaches have applications outside of proteomics, and are applicable to analytes more generally. For example, these approaches may be used in the context of metabolomics (e.g. for cancer screening and diagnosis, metabolic disorder profiling, or biofuel generation and use), environmental analysis (e.g. $CO_2$ monitoring or elemental analysis of heavy metal leaching), pharmaceutical analysis, and forensic analysis.

Nonetheless, all of the known approaches described above (for peptides, and for analytes more generally) rely on heuristic scoring criteria. In recent times there has been heavy investment in designing scores (or features) that separate 'correct' identifications from 'incorrect' identifications (a process termed 'feature engineering'). However, as they are subjective, these scores may still be prone to error in certain situations. For example, the subjective process of feature engineering can result in too many inadvertently generated co-linear scores, and potentially poor identification(s).

It would thus be desirable to provide systems and methods for identifying one or more analytes represented in (chimeric) mass spectrometry data which do not rely on feature engineered heuristic scores.

## Summary of the invention

It is an aim of the invention to provide improved systems and methods for identification of analytes (or molecular species) in mass spectrometry data where multiple analytes (or molecular species) may be present without the limitations of the systems of the prior art. Whilst such systems and methods may be particularly advantageous for use in the field of proteomics for identification of peptides, it will be appreciated from the discussion above and below that they are not limited to such a field and are generally applicable to mass spectrometry experiments.

In the present invention new methods and systems for identifying one or more analytes (or molecular species) represented in (chimeric) mass spectrometry data are proposed. More specifically, the invention provides a method for identifying one or more analytes (or molecular species) represented in (chimeric) mass spectrometry data using a machine learning model trained to determine an optimal scoring function for matching candidate molecular sequences to mass spectrometry data (e.g. a mass spectrum). The machine learning model is trained via 'end-to-end representation learning' to determine the optimal scoring function and does not rely on feature engineered heuristic scores.

In a first aspect, there is provided a (computer-implemented) method of identifying (or for identifying) one or more analytes represented in mass spectrometry data (such as or in the form of a mass spectrum), the method comprising:

obtaining a set of candidate molecular sequences for the mass spectrometry data (or mass spectrum), wherein each candidate molecular sequence corresponds to a respective candidate analyte; transforming the mass spectrometry data (or mass spectrum) into a mass spectrometry data vector (or mass spectrum vector) in a latent (or vector) space; transforming each candidate molecular sequence into a respective candidate molecular sequence vector in the latent (or vector) space; determining (or identifying or generating) a set of analyte coefficients for the set of candidate molecular sequences, based on the mass spectrometry data vector (or mass spectrum vector) and the candidate molecular sequence vectors (wherein the set of analyte coefficients directly indicate one or more analytes represented in the mass spectrometry data and the relative abundances of those one or more analytes); providing (or generating or outputting), for one or more of the candidate molecular sequences, a respective indication of a match (or presence) in the mass spectrometry (or mass spectrum) data, (directly) based at least in part on the set of analyte coefficients.

Each analyte coefficient may be determined based at least in part on a respective projection of (or relationship between, or correlation in the latent space between, or distance in the latent space between) the mass spectrometry (or mass spectrum) vector and the respective candidate molecular sequence vector.

Transforming the mass spectrometry (or mass spectrum) data may be carried out using a first trained neural network, and wherein transforming the set of candidate molecular sequences is carried out using a second trained neural network.

The first trained neural network and the second trained neural network may be (jointly) trained using a common loss function to output (or provide) vectors in the latent (or vector) space.

The common loss function may comprise one of: a) a projection of a mass spectrometry data vector generated by the first neural network on one or more corresponding molecular sequence vectors generated by the second neural network; and b) a projection of a candidate molecular sequence vector generated by the second neural network on a corresponding mass spectrometry data vector generated by the first neural network.

The first trained neural network may be a transformer and/or the second trained neural network may be a transformer.

The one or more analytes may comprise one or more peptides.

In a second aspect, there is provided a (computer-implemented) method of training a machine learning (or artificial intelligence) system to identify (or determine) one or more analytes represented in mass spectrometry (such as or in the form of a mass spectrum) data, the method comprising: providing (or generating) a training set comprising a plurality of items of (chimeric) mass spectrometry (or mass spectrum) data and, for each item of mass spectrometry (or mass spectrum) data a respective set of molecular sequences of analytes present in the mass spectrometry (or mass spectrum) data and a respective set of analyte coefficients; jointly training a first neural network and a second neural network using (or based on or from or with) the training set; wherein the first neural network takes as input (or receives as input or inputs or is configured to input) mass spectrometry (or mass spectrum) data and provides as output (or outputs or is configured to output) a mass spectrometry (or mass spectrum) data vector, and the second neural network takes as input a molecular sequence and provides as output (or outputs or is configured to output) a molecular sequence vector, wherein the step of jointly training optimizes the first and the second neural networks using a common loss function which comprises a similarity metric (or distance metric) between a mass spectrometry (or mass spectrum) data vector, a molecular sequence vector and the respective analyte coefficients.

The first neural network and the second neural network may share a common latent space.

The method may further comprise a step of augmenting (or altering or modifying or expanding) the training set prior to jointly training the first neural network and the second neural network.

The augmenting may comprise: generating one or more additional molecular sequences that are similar to (but not the same as) one of the molecular sequences of analytes present in the mass spectrometry data; and including (or adding) the one or more additional molecular sequences in (or into) the training set as (negative) examples of (or data representing) molecular sequences of analytes not present in the mass spectrometry data.

The one or more analytes may comprise one or more peptides; and the generating each additional molecular sequence may then comprise generating a first sequence that is (substantially) identical to the one of the molecular sequences of a peptide (or peptides) present in (or contributing to) the mass spectrometry (or mass spectrum) data and then reversing (the order of or inverting) the generated first sequence except for the last (or final or end) amino acid.

In a third aspect there is provided, a system arranged to carry out a method outlined above and/or described herein.

In a fourth aspect there is provided, a computer program which, when executed by one or more processors, causes the one or more processors to carry out a method outlined above and/or described herein.

In a fifth aspect there is provided, a computer-readable medium storing a computer program as outlined above.

That is, the invention also provides one or more computer programs suitable for execution by one or more processors, such computer program(s) being arranged to put into effect the methods outlined above and described herein. The invention also provides one or more computer readable media, and/or data signals carried over a network, which comprise (or store thereon) such one or more computer programs.

The invention also provides apparatus corresponding to, and comprising elements, modules or components arranged to put into effect the above methods for example one or more various suitably configured computing devices such as those described previously.

## Brief description of the drawings

Embodiments of the invention will now be described, by way of example only, with reference to the accompanying drawings in which:

Figure 1a schematically illustrates an example of a computer system which may be used in the invention.

Figure 1b schematically illustrates a system for analysing a sample using tandem mass spectrometry.

Figure 1c schematically illustrates example mass spectrometry data, such as may be produced by the system of figure 1b.

Figure 2 schematically illustrates a situation in which a machine learning system in accordance with the invention is used to identify the analytes represented in (chimeric) mass spectrometry data.

Figure 3a schematically illustrates an example system for training a machine learning system to identify one or more analytes represented in mass spectrometry data.

Figure 3b schematically illustrates an example method of training a machine learning system to identify one or more analytes represented in mass spectrometry data.

Figure 4a schematically illustrates an example system for identifying one or more analytes represented in mass spectrometry data.

Figure 4b schematically illustrates method of identifying one or more analytes represented in (chimeric) mass spectrometry data.

## Detailed description of embodiments of the invention

In the description that follows and in the figures, certain embodiments of the invention are described. However, it will be appreciated that the invention is not limited to the embodiments that are described and that some embodiments may not include all of the features that are described below. It will be evident, however, that various modifications and changes may be made herein without departing from the broader spirit and scope of the invention as set forth in the appended claims.

### General computer system:

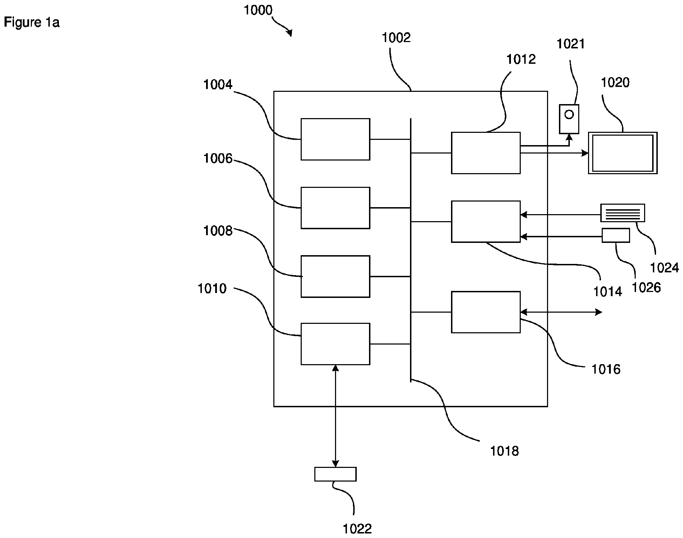

**Figure 1a** schematically illustrates an example of a computer system 1000. The system 1000 comprises a computer 1002. The computer 1002 comprises: a storage medium 1004, a memory 1006, a processor 1008, an interface 1010, a user output interface 1012, a user input interface 1014 and a network interface 1016, which may be linked together over one or more communication buses 1018.

The storage medium 1004 may be any form of non-volatile data storage device such as one or more of a hard disk drive, a magnetic disc, a solid-state-storage device, an optical disc, a ROM, etc. The storage medium 1004 may store an operating system for the processor 1008 to execute in order for the computer 1002 to function. The storage medium 1004 may also store one or more computer programs (or software or instructions or code).

The memory 1006 may be any random access memory (storage unit or volatile storage medium) suitable for storing data and/or computer programs (or software or instructions or code).

The processor 1008 may be any data processing unit suitable for executing one or more computer programs (such as those stored on the storage medium 1004 and/or in the memory 1006), some of which may be computer programs according to embodiments of the invention or computer programs that, when executed by the processor 1008, cause the processor 1008 to carry out a method according to an embodiment of the invention and configure the system 1000 to be a system according to an embodiment of the invention. The processor 1008 may comprise a single data processing unit or multiple data processing units operating in parallel, separately or in cooperation with each other. The processor 1008, in carrying out data processing operations for embodiments of the invention, may store data to and/or read data from the storage medium 1004 and/or the memory 1006.

The interface 1010 may be any unit for providing an interface to a device 1022 external to, or removable from, the computer 1002. The device 1022 may be a data storage device, for example, one or more of an optical disc, a magnetic disc, a solid-state-storage device, etc. The device 1022 may have processing capabilities - for example, the device may be a smart card. The interface 1010 may therefore access data from, or provide data to, or interface with, the device 1022 in accordance with one or more commands that it receives from the processor 1008.

The user input interface 1014 is arranged to receive input from a user, or operator, of the system 1000. The user may provide this input via one or more input devices of the system 1000, such as a mouse (or other pointing device) 1026 and/or a keyboard 1024, that are connected to, or in communication with, the user input interface 1014. However, it will be appreciated that the user may provide input to the computer 1002 via one or more additional or alternative input devices (such as a touch screen). The computer 1002 may store the input received from the input devices via the user input interface 1014 in the memory 1006 for the processor 1008 to subsequently access and process, or may pass it straight to the processor 1008, so that the processor 1008 can respond to the user input accordingly.

The user output interface 1012 is arranged to provide a graphical/visual and/or audio output to a user, or operator, of the system 1000. As such, the processor 1008 may be arranged to instruct the user output interface 1012 to form an image/video signal representing a desired graphical output, and to provide this signal to a monitor (or screen or display unit) 1020 of the system 1000 that is connected to the user output interface 1012. Additionally or alternatively, the processor 1008 may be arranged to instruct the user output interface 1012 to form an audio signal representing a desired audio output, and to provide this signal to one or more speakers 1021 of the system 1000 that is connected to the user output interface 1012.

Finally, the network interface 1016 provides functionality for the computer 1002 to download data from and/or upload data to one or more data communication networks.

It will be appreciated that the architecture of the system 1000 illustrated in figure 1a and described above is merely exemplary and that other computer systems 1000 with different architectures (for example with fewer components than shown in figure 1a or with additional and/or alternative components than shown in figure 1a) may be used in embodiments of the invention. As examples, the computer system 1000 could comprise one or more of: a personal computer; a server computer; a mobile telephone; a tablet; a laptop; a television set; a set top box; a games console; other mobile devices or consumer electronics devices; etc. Additionally, it is possible that some components of the computer system 1000 are not located in the computer 1002 and are, instead, part of a computer network connected to the computer 1002 via the network interface 1016. Additionally or alternatively, the computer system 1000 may comprise multiple computers 1002, e.g. in a network of computers such as a cloud system of computing resources.

**Mass spectrometry and mass spectrums:**

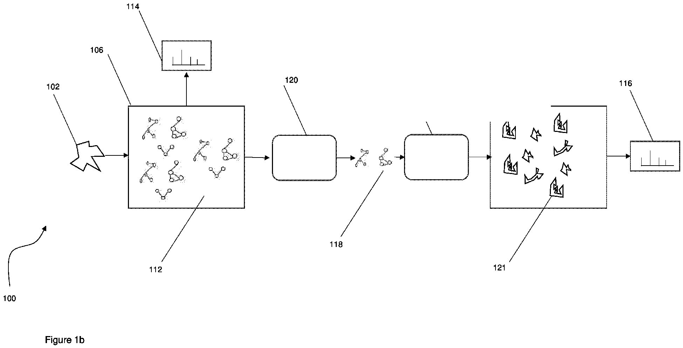

**Figure 1b** schematically illustrates a system 100 for analysing a sample 102 using tandem mass spectrometry. The system comprises an 'MS1' mass spectrometer 106, an isolation arrangement 120, a fragmentation arrangement 108, and an 'MS2' mass spectrometer 110.

Each of the 'MS1' and 'MS2' mass spectrometers comprise an ion source; a mass analyzer; and a detector. The ion source is used to convert a portion of the sample 102 into ions 112. The mass analyser is configured to separate the ions 112 according to their mass-to-charge (m/z) ratio. Each m/z value corresponds to one or more respective ionic species and is equal to the molecular mass of the respective ionic species divided by the absolute elemental charge of that respective ionic species. The detector is configured to detect the separated ions 112. The detection of the separated ions at the different m/z values allows for calculation of the abundances of ions present at each m/z ratio. In this way, each mass spectrometer (MS1/MS2) can be used to generate a mass spectrum 114, 116. The operation of mass spectrometers is well known in the art and not described further herein. It will be appreciated that any type of mass spectrometer may be used in system 100 as 'MS1' mass spectrometer 106 and an 'MS2' mass spectrometer 110. For example, each mass spectrometer 106, 110 may comprise a time of flight (TOF) mass spectrometer, a Fourier transform ion cyclotron resonance mass spectrometer (FT-ICRMS), an Orbitrap™ mass spectrometer and the like. It will be appreciated that the 'MS1' mass spectrometer 106 and the 'MS2' mass spectrometer 110 may be different types of mass spectrometer or different mass spectrometers (different pieces of hardware) of the same type. Alternatively, the 'MS1' mass spectrometer 106 and the 'MS2' mass spectrometer 110 may be performed on the same hardware (be the same mass spectrometer) operating in different modes (i.e. an 'MS1' mode and an 'MS2' mode respectively).

The isolation arrangement (or isolation means) 120 is configured to filter (or separate or isolate) separated ions by m/z value.

The fragmentation arrangement (or fragmentation means) 108 is configured to cause fragmentation of ions, for example by breaking bonds between them.

The sample 102 comprises one or more analytes, e.g. one or more peptides. The sample 102 may be obtained directly or may be obtained from the output of a pre-processing step or steps. For example, in bottom-up proteomics, where it is desired to identify proteins, the sample may be obtained by extracting protein (e.g. from tissue) and then applying pre-processing. This pre-processing may comprise proteolytic digestion using a peptidase, sample clean-up in order to remove unwanted salts or other reagents from the sample, offline fractionation (e.g. liquid chromatography) to decrease sample complexity, enrichment of specific classes of (modified) analytes using physicochemical properties or antibodies, and/or labelling using isobaric labelling reagents (e.g. tandem mass tags TMT, TMTpro, iTRAQ). The sample 102 is typically a liquid or a gas.

The sample 102 is input to the 'MS1' mass spectrometer 106 where the ion source of the 'MS1' mass spectrometer 106 is arranged to generate ions 112 of the one or more analytes in sample. These ions are typically gaseous ions. The ions may be generated by ionizing the sample (e.g. by electrospray ionization). The mass analyser of the 'MS1' mass spectrometer 106 is arranged to separate the ions of the ionized sample 102 as a function of m/z value. The detector of the 'MS1' mass spectrometer 106 is configured to detect the separated ions, measure the quantity (or relative abundance) of ions at each m/z value, and thereby obtain first mass spectrometry data 114 for the sample 102.

The separated ions 112 are output from the 'MS1' mass spectrometer and a subset of the separated ions 118 are input into the fragmentation arrangement 108. The subset 112 of the separated ions 118 are ions having a particular (or specific) m/z value or having an m/z value that falls within a particular (or specific) m/z value range. The subset of the separated ions 118 are isolated from the separated ions 112 by the isolation means 120. The isolation means 120 may be a mass filter. It will be appreciated that the isolation means and/or the fragmentation arrangement may be components of, or associated with, the 'MS1' mass spectrometer 106 and/or the 'MS2' mass spectrometer 110. For example, the 'MS2' mass spectrometer may have a mass filter arranged to switch to a mass-selective mode to select a subset 118 of the separated ions 112 having a m/z ratio within a predefined range for input to the fragmentation arrangement 108 also being a component of the 'MS2' mass spectrometer 118. The subset 118 of the separated ions may be thought of as forming a further sample. It will be appreciated that the further sample can alternatively or additionally be formed by selecting ions based on other physicochemical properties such as ion mobility.

The fragmentation arrangement (or fragmentation means) 108 receives the subset 118 of the separated ions 112 and fragments the ions of the subset 118 to produce a plurality of ion fragments 121. For example, the fragmentation may include physical breakage of the analyte ions 112, e.g. for peptides, breakage at the peptide bonds to produce amino acid fragments 121.

The ion fragments 121 are then input to the 'MS2' mass spectrometer 110 which operates in the same way as the 'MS1' mass spectrometer 106 but on ion fragments 121 rather than the sample 102 or the ions 112. In this way, the ion fragments 121 can be considered a second 'sample' 121. In particular, the 'MS2' mass spectrometer 110 is configured to separate the ions fragments 121 as a function of m/z value. The detector of the 'MS2' mass spectrometer 110 is configured to detect the separated ion fragments 121, measure the quantity (or relative abundance) of ion fragments 121 at each m/z value, and thereby obtain second mass spectrometry data 116 for the sample 102.

The second ('MS2') mass spectrometer 110 provides further structural information about the sample 102. This is achieved by allowing for discrimination of analytes present in the sample by virtue of expected patterns of their fragment ions and is beneficial in situations where accurate mass alone is not enough to determine an exact structure of an analyte. For example, where the one or more analytes comprise one or more peptides, the amino acid fragments 121 of those peptides and the second mass spectrometry data 116 can be used in conjunction with the first mass spectrometry data 114 to identify the particular peptides (and hence protein(s)) present in the sample 102. Nonetheless, it will be appreciated that mass spectrometry can be performed without the 'MS2' mass spectrometer 110 (using the 'MS1' spectrometer 106 alone). Equally, it will be appreciated that further isolation, fragmentation and analysis stages, e.g. MS3, MS4, MS5... could be performed to achieve further structural information. These stages could be performed using additional mass spectrometer hardware or as additional operations on the same mass spectrometer hardware as used for the 'MS1' mass spectrometer 106 and/or the 'MS2' mass spectrometer 110. The methods and systems of the invention as described herein below are generally applicable to any of the mass spectrometry stages and modes of operation described above.

The mass spectrometry data 114,116 may be represented in the form of one or more m/z channels, each corresponding to a respective m/z value. Here, the mass spectrometry data 114,116 may also comprise one or more intensity values with each intensity value appearing for a respective m/z value (or channel). Each intensity value indicates a relative abundance (or quantity) of ionic species corresponding to the respective m/z value as measured by the respective mass spectrometer 106, 110.

The mass spectrometry data 114,116 may be represented in the form of a mass spectrum (e.g. a line graph) or a list comprising the m/z values (or channels) and their respective intensity values. The mass spectrometry data 114,116 may be represented in many other forms as will be known to the person skilled in the art, such as a transient signal produced by an Orbitrap™ mass spectrometer or other Fourier- transform mass spectrometer such as an ion cyclotron resonance (FT-ICR) spectrometer, ion flight times produced by a time of flight (TOF) mass spectrometer, and so on. It will be appreciated that the methods and systems described below can be used with any representation of mass spectrometry data 112 (or mass spectra) .

It will be appreciated that for each respective m/z ratio, there may be more than one ionic species detected. This is often the case for chimeric mass spectra. In practice, chimeric spectra frequently occur in both data dependent acquisition (DDA) experiments and data independent acquisition (DIA) experiments.

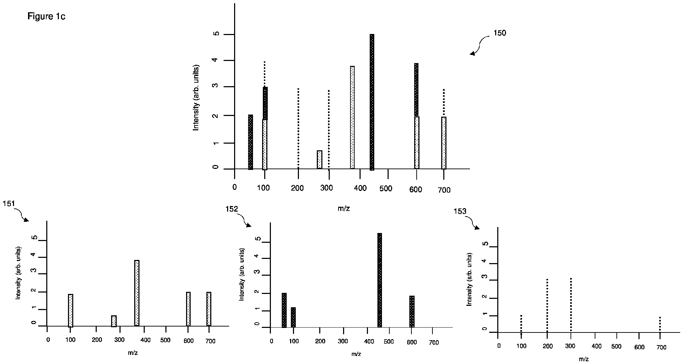

**Figure 1c** schematically illustrates four example mass spectrums 150, 151, 152, 153. Each mass spectrum 150, 151, 152, 153 represents mass spectrometry data generated by (or measured by) a mass spectrometer (specifically, the detector of a mass spectrometer), such as 'MS1' mass spectrometer 106 or 'MS2' mass spectrometer 110. As such, each mass spectrum 150, 151, 152, 153 may represent mass spectrometry data 114 or mass spectrometry data 116 described above.

Each mass spectrum 150, 151, 152, 153 is a graphical representation of mass spectrometry data, in the form of a histogram plot, and indicates m/z value on the x-axis and 'intensity' (or relative abundance) on the y-axis. That is, each mass spectrum 150, 151, 152, 152 may be a plot representing the intensity (or relative abundance) of ions at each m/z value (or mass channel).

Thus, at values of m/z for which the detector of the mass spectrometer that generated the mass spectrometry data detected analyte ions, there will be a non-zero value of intensity (or relative abundance). This non-zero value of intensity or relative abundance at a given m/z value is termed a 'peak' of a mass spectrum 150, 151, 152, 153.

Of these four example mass spectra 150, 151, 152, 153, mass spectrum 150 represents chimeric mass spectrometry data, and therefore comprises 'peaks' resulting from the presence of more than one analyte. That is, some 'peaks' are attributable (at least in part) to a first analyte and some peaks are attributable (at least in part) to a second analyte. Of course, it will be appreciated that if the first and second analyte in the chimeric spectrum both produce (or result in) ions generated at a first m/z value, then the 'peak' of mass spectrum 150 at that first m/z value will be attributable (at least in part) to the first and the second analyte. That is, in general, mass spectrum 150 comprises contributions from each of the analytes input to the mass spectrometer used to generate mass spectrum 150, e.g. sample 102 or ion fragments 121.

On the other hand, mass spectrum 151, mass spectrum 152, and mass spectrum 153 represent mass spectrometry data of an input (e.g. sample 102 or ion fragments 121) consisting of or derived from (substantially) a single analyte, and therefore all of their 'peaks' result from a single analyte. It will be appreciated that the input (e.g. sample or ion fragments 121) may not be completely pure, but the presence of other molecular species (e.g. peptides) in the sample would be at a level (or quantity or relative abundance) such that the impact of the other molecular species on the mass spectrum 15, 152, 153 is negligible.

In some examples, any or all of mass spectrum 150, mass spectrum 151, mass spectrum 152, and mass spectrum 153 may be generated by simulation (or numerical calculation.

As illustrated in figure 1c, mass spectrum 150 is the superposition of (or may be thought of as comprising the sum of) mass spectrum 151, mass spectrum 152 and mass spectrum 153. That is, figure 1c illustrates how a mass spectrum 150 of chimeric mass spectrometry data is the superposition (or sum of) the mass spectrums 151, 152, 153 of its constituent analytes. It will be appreciated that, in addition to the constituent analytes, mass spectrum 150 may comprise additional contributions to the intensity values at some (or each) value of m/z due to random noise and/or measurement error.

Thus, a mass spectrum such as mass spectrum 150 representing chimeric mass spectrometry data may be thought of in terms of a mathematical function representing intensity, $I$, as a function of mass-to-charge value (or ratio), $m/z$. For example, mass spectrum 150 may be represented as $I_0 = f(m/z)$ where:

$$I_0(m/z) = c_1 I_1(m/z) + c_2 I_2(m/z) + c_3 I_3(m/z) + \delta(m/z)$$

wherein $I_1(m/z)$, $I_2(m/z)$, and $I_3(m/z)$ represent mass spectrum 151, mass spectrum 152 and mass spectrum 153 respectively; $c_1$, $c_2$, and $c_3$ are scale factors (representing the relative abundance in the chimeric mass spectrometry data used to generate mass spectrum 150 of the analyte detected in the mass spectrometry data used to generate mass spectrum 151, mass spectrum 152, and mass spectrum 153 respectively); and $\delta(m/z)$ is a term representing measurement error and/or random noise in the mass spectrometer used to generate the mass spectrum 150 e.g. 'MS1' mass spectrometer 106 or 'MS2' mass spectrometer 110.

For mass spectrum 150, the scale factors $c_1$, $c_2$, and $c_3$ are all 1/3. That is, the input to the mass spectrometer used to generate mass spectrum 150 comprises equal amounts of each of the (single) analyte inputs used to generate mass spectrum 151, mass spectrum 152 and mass spectrum 153 respectively. However, it will be appreciated that other scale factors are possible.

It will also be appreciated that a mass spectrum may be generated from a mass spectrometer input comprising more than (or less than) three analytes. In this way, in general, a mass spectrum may be represented as

$$I(m/z) = \sum_{i=1}^{n} c_i I_i(m/z) + \delta(m/z)$$

, wherein, again, $I_i(m/z)$ is or represents a mass spectrum of the $i$th analyte present in the input used to generate the mass spectrum, $c_i$ is or represents a scale factor (or relative abundance) of the $i$th analyte present in the input used to generate the mass spectrum, $n$ is the total number of analytes present in the input used to generate the mass spectrum, and $\delta(m/z)$ is a term representing measurement error and/or random noise in the mass spectrometer used to generate the mass spectrum. For chimeric spectra, i > 1. Given chimeric spectra are combinations of pure spectra, the chimericity (i.e. the number of different contributing precursors) of a spectrum (or of mass spectrometry data) is a spectrum (or data) property.

**Example implementation of a machine learning system for identifying analytes represented in chimeric mass spectrometry data**

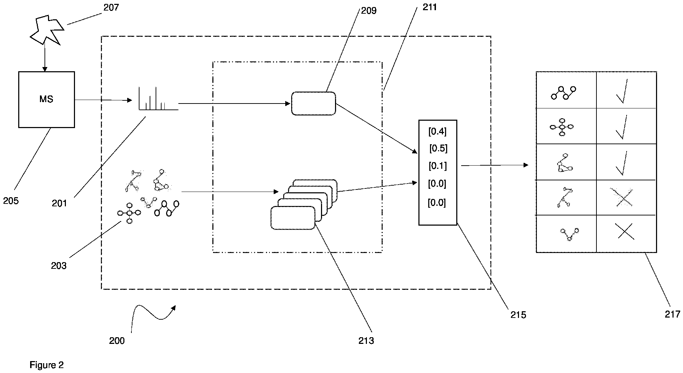

**Figure 2** schematically illustrates a situation in which a machine learning system 200 is used to identify analytes represented in a chimeric mass spectrum 201. The machine learning system 200 is trained to identify the analytes represented in the chimeric mass spectrum 201. In particular, the machine learning system 200 is arranged to embed both the chimeric mass spectrum 201 and candidate (or potential) molecular sequences 203 in a common latent space. In this way, both the scoring and identifying (or deconvolution) may be effected based on a distance measure between the embedded chimeric mass spectrum and the embedded candidate (or potential) molecular sequences. The machine learning system 200 is trained via 'end-to-end representation learning' to determine the embedding and does not rely on feature engineered heuristic scores or the like.

Specifically, the machine learning system 200 is configured to receive or otherwise obtain the mass spectrum 201. For example, the mass spectrum 201 may be received from a mass spectrometry instrument 205. The mass spectrum 201 may be, for example, mass spectrum 150, and the mass spectrometry instrument may be (or include), for example, 'MS1' mass spectrometer 106 or 'MS2' mass spectrometer 110. The mass spectrum 201 represents the analytes present in a sample 207 input to the mass spectrometer 205 used to produce the mass spectrum 201. The sample may be, for example, sample 102.

The machine learning system 200 is also configured to receive or otherwise obtain a set of candidate molecular sequences 203. Each candidate molecular sequence in the set 203 corresponds to a respective candidate analyte. Each candidate molecular sequence in the set 203 comprises (or is) an indication of a possible analyte represented in (or by) the mass spectrum 201, e.g., a possible analyte such as a peptide that may be present in (or derived from) the sample 207. That is, each analyte in the set 203 is a representation of (or an identifier of, or an indication of) the components (e.g. amino acids) that make up the structure of a (respective) candidate analyte. Each candidate molecular sequence may additionally comprise an indication of the structural arrangement of the components (e.g. amino acids) that make up the structure of the respective candidate analyte.

The machine learning system 200 is configured to allow for determination of which of the candidate molecular sequences in the set 203, if any, are represented in (or by) the mass spectrum 201.

In particular, the machine learning system 200 is configured to (or trained to) transform (or encode) the mass spectrum 201 into a mass spectrometry data vector 209 in a latent (or vector) space 211. The machine learning system 200 is also configured to (or trained to) transform (or encode) each candidate molecular sequence from the set 203 into a respective candidate molecular sequence vector 213 in the latent (or vector) space 211. The machine learning system 200 is further configured to determine a set of analyte coefficients 215 for the set of candidate molecular sequences 203, each analyte coefficient in the set of analyte coefficients 215 being based on the mass spectrometry data vector 209 and the respective candidate molecular sequence vector 213. For example, each analyte coefficient in the set of analyte coefficients 215 may be determined by (or calculated from or as) the dot (or scalar) product of the mass spectrometry data vector 209 and the respective candidate molecular sequence vector 213.

Each analyte coefficient in the set of analyte coefficients 215 represents a contribution of a different analyte to the mass spectrum 201, e.g. a first analyte coefficient represents a contribution of a first candidate analyte, and a second analyte coefficient represents a contribution of a second candidate analyte. Analyte coefficients corresponding to analytes not present in the mass spectrum 201 may be zero. Analyte coefficients corresponding to analytes present in the mass spectrum 201 may be non-zero. The total sum of the non-zero analyte coefficients may be 1, as illustrated in figure 2. In other words, the analyte coefficients 215 may directly indicate which analytes are represented in the mass spectrum 201 (via whether a coefficient is zero or non-zero) and, where represented, what their relative abundances are (via the magnitude of a non-zero coefficient).

The machine learning system 200 is further configured to provide, for one or more of the candidate molecular sequences, a respective indication 217 of a match in the mass spectrum 201, based at least in part on the set of analyte coefficients 215. For example, the indication 217 may be a binary 'yes'/'no' or 'tick'/'cross' indication associated with one or more of the candidate molecular sequences of the set 203. Here, a binary 'yes' or 'tick' indication may be provided for candidate molecular sequences 203 having a non-zero respective candidate analyte coefficient 215. In addition, a binary 'no' or cross' indication may be provided for candidate molecular sequences 203 having a zero respective candidate analyte coefficient 215. This allows for determination of the analytes represented in the mass spectrum 201 - e.g. those with a non-zero respective candidate analyte coefficient 215 are deemed represented (and present in the sample 207) and those with a zero respective candidate analyte coefficient 215 are deemed not represented (and not present in the same 207).

The specific details of the training of the machine learning system 200 will be described in more detail below. However, it will be understood that the projection of the mass spectrum 201 and the set of candidate molecular sequences 203 into the same latent space 211 that advantageously allows the machine learning system 200 to determine the set of analyte coefficients 215 directly, via 'end-to-end representation learning', without requiring feature engineered heuristic scores. This is because a) 'scoring' of the individual molecular sequences 203 as matches for the mass spectrum 201; and b) 'deconvolution' of the chimeric spectrum 201 into spectra of individual molecular sequences can be combined using a single loss function. The use of the single loss function is possible due to the use of the shared latent space 211.

In summary, machine learning system 200 essentially formulates deconvolution of chimeric spectra for molecular sequence identification as a machine learning problem which provides an end-to-end scoring and deconvolution method for chimeric spectra that does not rely on subjective/heuristic feature engineering.

**Training a machine learning system to identify analytes from mass spectrometry data**

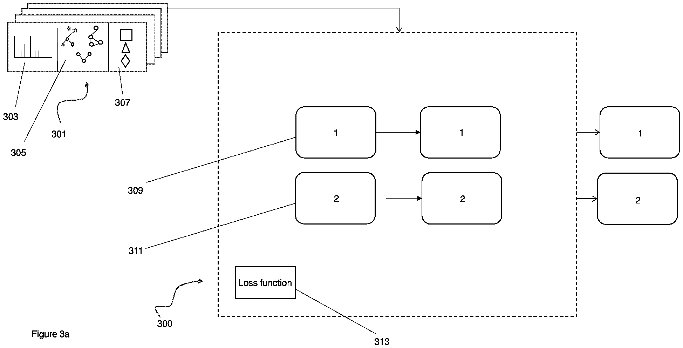

**Figure 3a** schematically illustrates an example system 300 for training a machine learning system to identify one or more analytes represented in mass spectrometry data. The mass spectrometry data may be chimeric mass spectrometry data. The mass spectrometry data may, for example, be represented by a mass spectrum such as mass spectrum 150, mass spectrum 151, mass spectrum 152, mass spectrum 153, or mass spectrum 201. The mass spectrometry data may, for example, be first mass spectrometry data 114 or second mass spectrometry data 116. The one or more analytes may comprise one or more peptides. The one or more analytes may consist of one or more peptides.

The system 300 may be implemented on a computer system such as computer system 1000.

The system is configured to receive a training set 301 comprising a plurality of items of chimeric mass spectrometry data 303 and, for each item of mass spectrometry data 303 a respective set of molecular sequences 305 of analytes present in the mass spectrometry data 303 and a respective set of analyte coefficients 307.

The chimeric mass spectrometry data 303 may be represented by a mass spectrum such as mass spectrum 150. However, as will be appreciated, the chimeric mass spectrometry data 303 may be represented in many other ways, such as a table or tables, a vector or vectors, a number of mass channels each with an associated m/z value and a corresponding intensity, a transient signal produced by an Orbitrap™ mass spectrometer or other Fourier- transform mass spectrometer such as an ion cyclotron resonance (FT-ICR) spectrometer, ion flight times produced by a time of flight (TOF) mass spectrometer, and so on. The chimeric mass spectrometry data may, for example, be first mass spectrometry data 114 or second mass spectrometry data 116.

The respective set of molecular sequences 305 for each item of mass spectrometry data represent (or may comprise a representation of) one or more of the analytes present in that (item of) mass spectrometry data 303. The respective set of molecular sequences 305 for each item of mass spectrometry data may represent (or may comprise a representation of) each analyte (or all analytes) present in that (item of) mass spectrometry data 303. The respective set of molecular sequences 305 for each item of mass spectrometry data may comprise or consist of representations of each analyte (or all analytes) present that (item of) mass spectrometry data 303. Each molecular sequence 305 in the respective set is a representation of (or an identifier of, or an indication of) the components that make up the structural sequence of a (respective) analyte in that (item of) mass spectrometry data 303. For example, where the analytes are peptides, each molecular sequence 305 in the respective set is a peptide sequence representing the amino acids that make up the structural sequence of a (respective) peptide in that (item of) mass spectrometry data 303. Each molecular sequence 305 may additionally comprise an indication of the structural arrangement of the components that make up the structure of the respective analyte in that (item of) mass spectrometry data 303. There are a number of different ways that the molecular sequences 305 may be represented, as would be appreciated by the skilled person. For example, each molecular sequence 305 in the respective set may be represented as an expressed sequence tag, as described at J Parkinson et al., Expressed sequence tags: an overview, Methods Mol Biol. 2009:533:1-12. doi: 10.1007/978-1-60327-136-3_1, the entire content of which is incorporated herein by reference, and which can be accessed from https://link.springer.com/protocol/10.1007/978-1-60327-136-3 1 . Each molecular sequence 305 in the respective set may additionally or alternatively be represented as a code, as described at https://www.ebi.ac.uk/pdbe/docs/roadshow tutorial/msdtarget/AAcodes.html and https://www.ncbi.nlm.nih.gov/pmc/articles/PMC4665559/table/diagnostics-04-00140-t001/ both of which are incorporated herein by reference in their entirety. In such a code, where the analyte is a peptide, the constituent amino acids may, for example, be represented by their three-letter symbol or their one-letter symbol. Each molecular sequence 305 in the respective set may additionally or alternatively be represented graphically, as described at https://www.britannica.com/science/amino-acid/Amino-acid-reactions , the entire content of which is incorporated herein by reference. Each molecular sequence may additionally or alternatively be represented as raw data or encoded data, as described at https://www.nature.com/articles/s41467-021-24496-9 the entire content of which is incorporated herein by reference. Where a molecular sequence 305 is a peptide sequence, that peptide sequence may additionally or alternatively be represented as one-hot encoding of amino acids, as described at S Gessulat et al., Prosit: proteome-wide prediction of peptide tandem mass spectra by deep learning, Nat Methods. 2019 Jun;16(6):509-518. doi: 10.1038/s41592-019-0426-7. Epub 2019 May 27, the entire content of which is incorporated herein by reference, and which can be accessed from https://pubmed.ncbi.nlm.nih.gov/31133760/. Each molecular sequence may additionally or alternatively be represented using chemical compositions, chemical structures, molecular graphs etc as would be familiar to the skilled person. Each molecular sequence may additionally or alternatively be represented as standardized descriptors from which a chemical structure may be generated (e.g. InChl or SMILES descriptors). InChl (International Chemical Identifier) descriptors are described at S Heller et. al. "InChl, the IUPAC International Chemical Identifier" Journal of Cheminformatics volume 7, Article number: 23 (2015) which can be accessed from https://jcheminf.biomedcentral.com/articles/10.1186/s13321-015-0068-4, the entire content of which is incorporated herein by reference in their entirety. SMILES (simplified molecular-input line-entry system) descriptors are described at Wigh DS, Goodman JM, Lapkin AA. A review of molecular representation in the age of machine learning. WIREs Comput Mol Sci. 2022, https://doi.org/10.1002/wcms.1603 the entirety of which is incorporated herein by reference in their entirety.

The respective set of analyte coefficients 307 comprise one or more coefficients (or values) representing one or more of the analytes present in that (item of) mass spectrometry data 303. The respective set of analyte coefficients may comprise coefficients representing each (or all) of the analytes present in that (item of) mass spectrometry data 303. Each analyte coefficient 307 in the set may correspond to one (or more) respective molecular sequence 305 of the set of molecular sequences 305 for that item of mass spectrometry data 303. The respective set of analyte coefficients 307 may be represented as a table, or a vector, or a collection of numerical values. However, it will be appreciated that the coefficients may be represented in other ways, such as with alphanumeric values.

In one example, each respective set of analyte coefficients 307 may be a vector. The vector may have a length (or number of elements) corresponding to (or equal to) the number of molecular sequences in the respective corresponding set of molecular sequences for the respective (item of) mass spectrometry data 303. Each element in the vector may correspond to a respective molecular sequence 305 in the respective corresponding set of molecular sequences for the respective (item of) mass spectrometry data 303. The vector may include a zero (or null) element (or coefficient) in a position corresponding to a molecular sequence of the set 305 if (or to indicate or to represent that) that molecular sequence is not present in the mass spectrometry data 303. The vector may include a non-zero element (or coefficient) in a position corresponding to a molecular sequence of the set 305 if (or to indicate or to represent that) that molecular sequence is present in the mass spectrometry data 303. For example, the vector may be a vector of 0s and 1s, with each 1 representing a analyte molecular sequence in the set 305 that is present in the respective mass spectrometry data 303 and each 0 representing a molecular sequence in the set 305 that is not present in the respective mass spectrometry data 303. In another example, the vector may be a vector of zero values and non-zero values. Each non-zero value may represent a molecular sequence in the set 305 that is present in the respective mass spectrometry data 303 and the relative abundance of that molecular sequence in the mass spectrometry data 303. Each zero value may represent a molecular sequence in the set 305 that is not present in the respective mass spectrometry data 303. A higher (or greater or larger) non-zero value may represent a higher (or greater or larger) relative abundance. The total sum of the non-zero analyte coefficients may be 1. In other words, the analyte coefficients 307 may (or may be configured to) directly indicate (or identify) which analytes are represented in the mass spectrometry data 303 (via whether a coefficient is zero or non-zero) and, where represented, what their relative abundances are (via the magnitude of a non-zero coefficient).

The training set 301 comprises a plurality of items of chimeric mass spectrometry data 303. Each item of chimeric mass spectrometry data in the training set 301 (and its respective (or corresponding) set of molecular sequences of analytes present in the mass spectrometry data and respective (or corresponding) set of analyte coefficients) may be represented in the same way or may be represented in different ways.

The training set 301 may be, for example, received from a user interface such as user input interface 1014 or a device such as device 1022. The training set 301 may be, for example, received from memory 1006 of the computer 1002 that the system 300 is implemented (or performed) on. The training set 301 may be, for example, received from a storage medium 1004 of the computer 1002 that the system 300 is implemented (or performed) on.

The system 300 is configured to use the training set 301 to jointly train a first neural network 309 and a second neural network 311. The joint training may be training such that the first neural network 309 and the second neural network 311 share a common latent space. That is, the machine learning system that the system 300 is for training may comprise the first neural network 309 and the second neural network 311. The first neural network 309 and the second neural network 311 are (or implement) machine learning models. The first neural network 309 and the second neural network 311 may be deep artificial neural networks, which would be familiar to the skilled person and which are therefore not described further herein. It will be appreciated that the first neural network 309 and the second neural network 311 may be implemented as different operations on a single neural network architecture. That is, the first neural network 309 and the second neural network 311 are described as two neural networks in the sense that there are two inputs and two outputs, but they may both be implemented on the single neural network architecture.

The first neural network 309 takes (or is configured to take or receive) as input mass spectrometry data such as mass spectrometry data 303 and provides (or is configured to provide) as output a mass spectrometry data vector.

The second neural network 311 takes (or is configured to take or receive) as input a molecular sequence such as from the sets of molecular sequences 305 and provides (or is configured to provide) as output an molecular sequence vector.

The mass spectrometry data vector and the molecular sequence vector may be the same length. The mass spectrometry data vector and the molecular sequence vector may be output to a common latent space (the common latent space shared by the first neural network and the second neural network). That is, the first neural network 309 and the second neural network 311 may be independent neural networks which together transform one or more items of mass spectrometry data and one or more molecular sequences into one or more vector representations in a shared latent space.

One or both of the first neural network 309 and the second neural network 311 may be an encoder or a decoder (or an autoencoder or autodecoder- which would be familiar to the skilled person and which is therefore not described further herein). For example, the first neural network 309 and the second neural network 311 may both be encoders or decoders (or autoencoders or autotdecoders) having a network architecture comprising: an input embedding; three stacked regularized transformer layers with 16 attention heads (as described at Vaswani, A., et al., Attention Is All You Need. arXiv, 2017, which can be accessed from https://arxiv.org/abs/1706.03762, and which is incorporated herein by reference in its entirety); 16 key dimensions; a GRU layer (gated recurrent unit layer - see for example Chung, J., et al., Empirical Evaluation of Gated Recurrent Neural Networks on Sequence Modeling, arXiv, 2014, which can be accessed from https://arxiv.org/abs/1412.3555 and which is incorporated by reference in its entirety) with 128 memory dimensions; and a final dense layer projecting the GRU output from 128 dimensions to 32.

In other examples, the GRU layer may not be present, and the use of transformer layers without a GRU layer may, in fact, be advantageous as described at Vaswani, A., et al., Attention Is All You Need. arXiv, 2017, which can be accessed from https://arxiv.org/abs/1706.03762 , and which is incorporated herein by reference in its entirety.

Where the second neural network 311 is an encoder and where the analytes are peptides, the second neural network 311 may be configured to use positional embedding on a set of unique integer tokens for each amino acid in input molecular sequences. For example, the second neural network 311 may use a sinusoidal embedding with 256 channels (as described at Yilmaz, M., et al., De novo mass spectrometry peptide sequencing with a transformer model. bioRxiv, 2022: p. 2022.02.07.479481, which can be accessed from https://doi.org/10.1101/2022.02.07.479481 and which is incorporated herein by reference in its entirety).

Each of the first neural network 309 and the second neural network 311 may be defined by hyperparameters, and the hyperparameters may be chosen based, at least in part, on: a) limitations of the graphical processing unit (GPU) of the system used to train the machine learning system; and/or b) the impact of the hyperparameters on the performance of the first neural network 309 and/or the second neural network.

In general, using a larger neural network (e.g. more stacked Transformer layers) for one or more of the first neural network 309 and the second neural network 311 improves of that (or those) neural networks but requires a GPU with more memory for training.

In some examples, the transformer layers may use (or include or comprise or implement) weight decay (e.g. normalisation of neural network weights, such as L2-norm normalisation of weights, as described at Zhang G, et al., Three Mechanisms of Weight Decay Regularization, arXiv, 2018, which can be accessed https://doi.org/10.48550/ar Xiv.1810.12281 and which is incorporated herein by reference in its entirety. The use of weight decay in transformer layers improves convergence of the first neural network 309 and the second neural network 311.

The system 300 is configured (or arranged) to jointly train the first neural network 309 and the second neural network 311 to optimize (or update) the first neural network 309 and the second neural network 311 using a common loss function 313 which comprises a similarity metric between a mass spectrometry data vector (such as a mass spectrometry data vector output from the first neural network 309), a molecular sequence vector (such as a molecular sequence vector output from the second neural network 311) and the respective analyte coefficients 307.

The objective of the training is that the first neural network 309 and the second neural network 311 learn to project spectra and input molecular sequences into a shared latent representation that enables (or makes it possible or easier to) derive the contributions of a set of candidate molecular sequences, e.g. the set of molecular sequences 305, to a given item of mass spectrometry data, e.g. the mass spectrometry data 303.

Here the loss function 313 is designed to evaluate how well the latent space vectors (i.e. the mass spectrometry data vectors and the molecular sequence vectors) correlate to the analyte coefficients 307 of the training dataset 301. That is, the projection into the latent space allows for a comparison to be made between (a function of) the latent space vectors and their respective analyte coefficients 307.

The common loss function 313 may be, for example, a function that takes as input one or more mass spectrometry data vector (such as a mass spectrometry data vector output from the first neural network 309) and one or more respective (or corresponding) molecular sequence vector (such as a molecular sequence vector output from the second neural network 311) and for each respective item of mass spectrometry data, the common loss function may generate a predicted analyte coefficient based on a) the respective mass spectrometry data vector, and b) the respective molecular sequence vector. The common loss function may generate the predicted analyte coefficient based directly on the a) the respective mass spectrometry data vector, and b) the respective molecular sequence vector. For example, the common loss function 313 may generate each predicted analyte coefficient by multiplying (or generating the dot or scalar product of or between) a) the respective mass spectrometry data vector, and b) the respective molecular sequence vector. The common loss function 313 may then calculate (or define) a similarity between each predicted analyte coefficient and its respective analyte coefficient 307 such that optimizing the first and the second neural network using the common loss function 313 increases (or maximises) the similarity between the predicted analyte coefficients and the respective analyte coefficients 307. In other words, the similarity metric minimizes the distance between the distributions of the predicted analyte coefficients and the respective analyte coefficients 307.

In this way, the common loss function $L(P,s,c)$ may be defined as:

$$L(P,s,c) = 1 - SA(P,s,c),$$

where $P$ is a set of molecular sequence vectors, $s$ is a respective set of mass spectrometry data vectors, $c$ is the respective analyte coefficients for the mass spectrometry data used to generate the respective set of mass spectrometry data vectors, and $SA$ is a similarity metric.

The similarity metric is a function and may, in some examples, be a normal spectral contrast angle distance metric, which is defined as:

$$SA(P,s,c) = 1 - (^{2\cos^{-1}(\hat{s} \cdot \hat{P})}/_{\pi}),$$

$$\hat{s} = {s}/{\sqrt{\sum_{i=0}^{n} s_i^2}} \text{ and } \hat{P} = {P}/{\sqrt{\sum_{i=0}^{m} P_i^2}}$$

where and $n$ and $m$ are the number of vector elements in $s$ and $P$ respectively. It will be appreciated that in some examples, $n = m$.

The use of a normal spectral contrast angle distance metric as the similarity metric is advantageous because it is order invariant and easy to mask out dimensions in the vectors that do not matter (e.g. if the vectors used are longer than required (fewer molecular sequences) and therefore are filled with null values). However, it will be appreciated that other order-invariant similarity metrics may have a similar effect.

The common loss function 313 may be, for example, received from a user interface such as user input interface 1014 or a device such as device 1022. The common loss function 313 may be, for example, received from memory 1006 of the computer 1002 that the system 300 is implemented (or performed) on. The common loss function 313 may be, for example, received from a storage medium 1004 of the computer 1002 that the system 300 is implemented (or performed) on.

The trained first neural network 309 and/or second neural network 311 may be output from system 300, for example via output interface 1012. The trained first neural network 309 and/or second neural network 311 may additionally or alternatively be stored on storage medium 1004 or in memory 1006. The trained first neural network 309 and/or second neural network 311 may additionally or alternatively be transmitted over network interface 1016.

The trained first neural network 309 and/or second neural network 311 may be implemented on (or run on or with or applied to) 'test' data to identify one or more analytes represented in chimeric mass spectrometry data. Such test data may comprise a chimeric mass spectrum such as mass spectrum 150 or chimeric mass spectrometry data 114 or second mass spectrometry data 116 as described in more detail below.

The system 300 above for training a machine learning system to identify one or more analytes represented in mass spectrometry data is advantageous because it does not require feature engineering. Instead, it exploits the idea that chimeric mass spectrometry data is made from combinations of 'pure' mass spectrometry data, and so the chimericity (e.g. the number of and relative abundance of individual analyte whose 'pure' mass spectrometry data make up the chimeric mass spectrometry data) of mass spectrometry data is a property of that data. This property can be learned and generalized using machine learning. Thus, using the training system 300 described above, an end-to-end machine learning model can be trained that can essentially 'deconvolute' chimeric spectra/mass spectrometry data and assign coefficients representing the contribution of different analytes per spectrum/item of mass spectrometry data.

It will be appreciated that the first neural network 309 and the second neural network 311 could each individually or both together be substituted for any other type of machine learning model, as would be appreciated by the person skilled in the art.

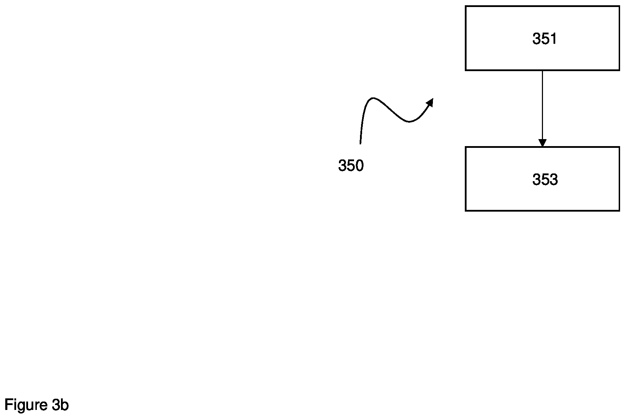

**Figure 3b** schematically illustrates an example method 350 of training a machine learning system to identify one or more analytes represented in mass spectrometry data. The method 350 may be implemented on (or with or using) system 300. That is, the machine learning system that the method 350 is for training may comprise the first neural network 309 and the second neural network 311 (which, as described above, may be implemented on the same or different neural network architectures).

The mass spectrometry data may, for example, be represented by a mass spectrum such as mass spectrum 150, mass spectrum 151, mass spectrum 152, mass spectrum 153, or mass spectrum 201. The mass spectrometry data may,

13

for example, be first mass spectrometry data 114 or second mass spectrometry data 116.

The mass spectrometry data may be chimeric mass spectrometry data.

The method 350 may be implemented on a computer system such as computer system 1000.

In method 350, a first step 351 comprises providing a training set comprising a plurality of items of chimeric mass spectrometry data and, for each item of mass spectrometry data a respective set of molecular sequences of analytes present in the mass spectrometry data and a respective set of analyte coefficients. The training set may be training set 301.

The providing the training set may comprise generating the training set or receiving the training set.

The training set may be, for example, received from a user interface such as user input interface 1014 or a device such as device 1022. The training set may be, for example, received from memory 1006 of the computer 1002 that the system 300 is implemented (or performed) on. The training set may be, for example, received from a storage medium 1004 of the computer 1002 that the system 300 is implemented (or performed) on.

The training set may be generated by or using (or may have been generated by, if received) various methods that would be appreciated by the person skilled in the art. The training set may be generated from training data. For example, the training set may be generated by combining experimental non-chimeric spectra in silico (the training data comprising the combined experimental non-chimeric spectra). Additionally or alternatively, the training set may be generated by predicting fragment spectra with models such as pDeep (as described in Zhou, X-X., et al., pDeep: Predicting MS/MS Spectra of Peptides with Deep Learning. Anal. Chem., 2017. 89(23): p. 12690-12697, which can be accessed from https://doi.org/10.1021/acs.analchem.7b02566 and which is incorporated herein by reference in its entirity) or Prosit (as described at Gessulat, S., et al., Prosit: proteome-wide prediction of peptide tandem mass spectra by deep learning. Nat. Methods, 2019. 16: p. 509-518, which can be accessed from https://pubmed.ncbi.nlm.nih.gov/31133760/ and which is incorporated herein by reference in its entirety) and combining the spectra for multiple analytes (the training data comprising the combined spectra). Additionally or alternatively, the training set may be generated using experimental data where the chimericity has been determined in advance (the training data comprising the experimental data). Such suitable experimental data may include data from (or based on) one of more of the following, all of which are incorporated herein by reference in their entirety: a) J B. Muller et. al. "The proteome landscape of the kingdoms of life", Nature, 582, 592-596 (2020), which can be accessed from https://doi.org/10.1038/s41586-020-2402-x; b) P E. Geyer et al. "Plasma Proteome Profiling to detect and avoid sample-related biases in biomarker studies", EMBO Mol Med (2019), which can be accessed from https://doi.org/10.15252/emmm.201910427; c) Y Bian et al. "Robust, reproducible and quantitative analysis of thousands of proteomes by micro-flow LC-MS/MS" Nat Commun 11, 157 (2020), which can be accessed from https://doi.org/10.1038/s41467-019-13973-x; d) D B. Bekker-Jensen et al. "An Optimized Shotgun Strategy for the Rapid Generation of Comprehensive Human Proteomes" 2017, which can be accessed from https://doi.org/10.1016/j.cels.2017.05.009 ; e) C D. Kelstrup et al. "Performance Evaluation of the Q Exactive HF-X for Shotgun Proteomics" J. Proteome Res. 2018, 17, 1, 727-738, which can be accessed from https://doi.org/10.1021/acs.jproteome.7b00602; f) K Sharma et. al "Ultradeep Human Phosphoproteome Reveals a Distinct Regulatory Nature of Tyr and Ser/Thr-Based Signaling" 2014 which can be accessed from https://doi.org/10.1016/j.celrep.2014.07.036; g) K Krug et al. "Proteogenomic Landscape of Breast Cancer Tumorigenesis and Targeted Therapy" 2020, which can be accessed from https://doi.org/10.1016/j.cell.2020.10.036; and h) A Stukalov et al. "Multilevel proteomics reveals host perturbations by SARS-CoV-2 and SARS-CoV" Nature 594, 246-252 (2021), which can be accessed from https://doi.org/10.1038/s41586-021-03493-4. An example training dataset 301 comprising all of these would be beneficial because it would be large and would comprise a diverse set of data dependent acquisition (DDA) and data independent acquisition (DIA) datasets from different mass spectrometry instruments and based on experiments from different parties. Where the respective set of analyte coefficients are numerical values configured to directly indicate (or identify) which analytes are represented in the mass spectrometry data (via whether a coefficient is zero or non-zero) and, where represented, what their relative abundances are (via the magnitude of a non-zero coefficient), the coefficients can be extracted directly from training data used to generate the training dataset. Additionally or alternatively, the training set may be generated using the search engine CHIMERYS (as described at https://www.biorxiv.org/content/10.1101/2024.05.27.596040v2 and EP4102509A1; METHOD AND APPARATUS FOR IDENTIFYING MOLECULAR SPECIES IN A MASS SPECTRUM. 2022, both of which are incorporated herein by reference in their entirety). For example, one way of generating a training set is to search for and then create a training set from (or including or comprising) a subset (e.g. brain tissue samples) of MS2 spectrum files from Wang, D., et al., A deep proteome and transcriptome abundance atlas of 29 healthy human tissues. Mol. Syst. Biol., 2019. 15(2): p. e8503 (which can be accessed from https://doi.org/10.15252/msb.20188503 and which is incorporated herein by reference in its entirety). In CHIMERYS the maximum number of candidate molecular (in this case, peptide) sequences that are considered per experimental spectrum can be configured. For example, to create the training set, this number may be set to 15.

In method 350, a second step 353 comprises jointly training (or training using a common loss function) a first neural network and a second neural network using the training set. The first neural network may be first neural network 309 and the second neural network may be second neural network 311. The first neural network takes as input mass spectrometry data and provides as output a mass spectrometry data vector, and the second neural network takes as input a molecular sequence and provides as output a molecular sequence vector. The step of jointly training optimizes (or updates) the first and the second neural networks using a common loss function which comprises a similarity metric between a mass spectrometry data vector, a molecular sequence vector and the respective analyte coefficients.

The common loss function may be common loss function 313. Thus, the common loss function may be, for example, a function that takes as input one or more mass spectrometry data vectors (such as a mass spectrometry data vector output from the first neural network 309) and one or more respective (or corresponding) molecular sequence vectors (such as a molecular sequence vector output from the second neural network 311) and for each respective item of mass spectrometry data, the common loss function may generate a predicted analyte coefficient based on a) the respective mass spectrometry data vector, and b) the respective molecular sequence vector. That is, in some examples, method 350 may be framed as a method of training a machine learning system to (directly) identify analytes represented in mass spectrometry data, the method comprising: providing a training set comprising a plurality of items of (chimeric) training mass spectrometry data and, for each item of training mass spectrometry data, a respective set of training molecular sequences of analytes present in the training mass spectrometry data and a respective set of training analyte coefficients; jointly training a first machine learning model and a second machine learning model using the training set; wherein the first machine learning model is configured to input mass spectrometry data and output a mass spectrometry data vector corresponding to the input mass spectrometry data, and the second machine learning model is configured to input a molecular sequence and output a molecular sequence vector corresponding to the molecular sequence; and wherein the step of jointly training comprises: for each respective item of training mass spectrometry data, generating a predicted analyte coefficient based on a) a respective training mass spectrometry data vector corresponding to that item of training mass spectrometry data, and b) a training molecular sequence vector corresponding to the training molecular sequence; and updating (or optimising) the first and second neural networks using a common loss function which comprises a similarity metric between the predicted analyte coefficients and the training analyte coefficients.

In method 350, the first neural network and the second neural network may share a common latent space. In this way, the method 350 may implement the training process using a single loss function.

In method 350, there may be a further optional step of augmenting the training set prior to jointly training the first neural network and the second neural network.

The augmenting may comprise generating one or more additional molecular sequences that are similar to one of the molecular sequences of analytes present in the mass spectrometry data of the training set; and including the one or more additional molecular sequences in the training set as examples molecular sequences of analytes not present in the mass spectrometry data. Where, as described above, each respective set of analyte coefficients is a vector, the including the one or more additional molecular sequences in the training set as examples of molecular sequences of analytes not present in the mass spectrometry data may comprise adding a 'zero' or 'null' value in an element of the vector representing the respective set of analyte coefficients at a position corresponding to the additional molecular sequence. Where the analyte is a peptide, the generating each additional molecular sequence may, for example, comprise: generating a first sequence that is identical to the one of the molecular sequences of analytes present in the mass spectrometry data and then reversing the generated first sequence except for the last amino acid.

More generally, generating each additional molecular sequence may, for example, comprise shuffling (or reordering or permuting) InChI or SMILES descriptors (or representations) of a molecular sequence of an analyte present in the mass spectrometry data to generate an additional molecular sequence of a similar-looking but non-present analyte (or molecular species). Additionally, or alternatively, generating each additional molecular sequence may, for example, comprise shuffling (or reordering or permuting) a chemical structure of an analyte present in the mass spectrometry data, and then generating, from the shuffled chemical structure, a corresponding additional molecular sequence of the shuffled (not-present) analyte. In either case, an additional optional step may be performed of checking whether the generated molecular sequence describes a chemically valid structure.

Where the analyte is a peptide, the generating each additional molecular sequence may, for example, additionally or alternatively comprise: generating a second sequence that is identical to the one of the molecular sequences of analytes present in the mass spectrometry data except for one amino acid in the generated second sequence which is different from the one of the molecular sequences.

In this way, the generating additional molecular sequences may allow for augmentation of the training set by hardest sample mining. That is, examples of molecular sequences not present in the mass spectrometry data may be generated or provided (e.g. as candidate sequences with a zero-coefficient in the respective set of analyte coefficients for that mass spectrometry data) that look most like positive examples (molecular sequences that are present in the mass spectrometry data). These negative additional molecular sequences can be generated from a sequence present in the mass spectrometry data. For example, where the analyte is a peptide, by reversing the sequence except for the last amino acid. Such reversing changes the mass spectrum fragmentation behaviour of the peptide but does not change the precursor mass of the peptide. In another example, where the analyte is a peptide, these negative additional molecular sequences can also or instead be generated by single amino acid-swaps from a peptide sequence present in the mass spectrometry data. However, the single amino acid swap augmentation may lead to less accurate coefficient predictions than the sequence reversal augmentation. The use of hardest sample mining may improve the performance of the trained machine learning system by increasing the accuracy of the trained machine learning system.

The augmenting may additionally or alternatively comprise: generating one or more modified molecular sequences, wherein generating each modified molecular sequence comprises generating a third sequence that is identical to the one of the molecular sequences of analytes present in the mass spectrometry data and then permuting the sequence order of the generated third sequence; and including each of the one or more modified molecular sequences in the training set as an example of a molecular sequence of an analyte present in the mass spectrometry data. This advantageously allows for provision of examples to the machine learning system to train the machine learning system such that the coefficients are order-invariant. As a result, the accuracy of the trained machine learning system is increased. Experiments have shown that such permutation produces a particularly significant performance increase for the method where the training set is small.

In method 350, the chimeric mass spectrometry data may represent at least three different analytes. That is, the chimeric mass spectrometry data may include contributions from at least three different analytes. This makes the training set more suitable for training the machine learning system on (or to learn) how to derive the analyte coefficients of analytes in complex and highly chimeric spectra. The choice of a minimum of three non-zero analyte coefficients was chosen to be low, to keep most spectra from the dataset. However, it will be appreciated that instead, for example, the chimeric mass spectrometry data may represent at least 2, 4, 5, 6, 7, 8, 9, 10, or 20 or more different analytes. Where the chimeric mass spectrometry data is represented as a mass spectrum, such as mass spectrum 150, this means that the mass spectrum includes peaks from at least three precursors (e.g. it is the superposition of at least 3 'pure' spectra such as mass spectrum 151, mass spectrum 152 and mass spectrum 153). To achieve this where, as described above, each respective set of analyte coefficients is a vector, method 350 may include a further optional step of modifying the training set to discard items of mass spectrometry data for which the respective set of analyte coefficients has fewer than three non-zero coefficients. This can be done manually or may be done in an automated manner, e.g. using scanning software.

Some training sets may include decoy sequences, depending on how and where they are obtained. Decoy sequences are molecular sequences that do not exist in nature and are described at J Elias et al., Target-Decoy Search Strategy for Mass Spectrometry-Based Proteomics, Methods Mol Biol. 2010; 604: 55-71. doi: 10.1007/978-1-60761-444-9_5, which is incorporated herein by reference in its entirety, and which can be accessed from https://link.springer.com/protocol/10.1007/978-1-60761-444-9 5. Such decoy sequences are also described at J Elias et al., Comparative evaluation of mass spectrometry platforms used in large-scale proteomics investigations Nat Methods 2, 667-675 (2005), which is incorporated herein by reference in its entirety, and which can be accessed from https://doi.org/10.1038/nmeth785. Decoy sequence generation for metabolites is described at X Wang et.al., "Target-Decoy-Based False Discovery Rate Estimation for Large-Scale Metabolite Identification", J Proteome Res. 2018, which can be accessed from https://doi.org/10.1021/acs.jproteome.8b00019 and which is incorporated herein by reference in its entirety.

In such cases, the training set may optionally be modified prior to jointly training the first neural network and the second neural network by removing (or excluding) the decoy sequences from the training set. In this way, bias in the trained machine learning system is reduced as compared to if the decoy sequences were retained. As a result, the accuracy of the trained machine learning system is increased.

It will be appreciated that there may be provided a system arranged to carry out method 350. It will also be appreciated that there may be provided computer program which, when executed by one or more processors, causes the one or more processors to carry out method 350. The one or more processors may comprise processor 1008. It will also be appreciated that there may be provided a computer-readable medium storing such a computer program.

**Identifying one or more analytes represented in chimeric mass spectrometry data:**

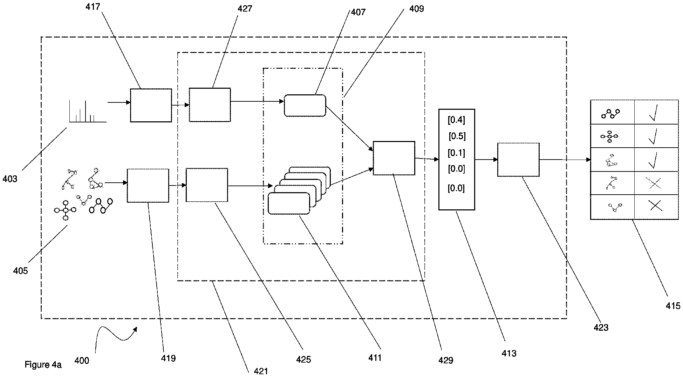

**Figure 4a** schematically illustrates an example system 400 for identifying one or more analytes represented in mass spectrometry data 403. The mass spectrometry data 403 may be chimeric mass spectrometry data. The mass spectrometry data may, for example, be represented by a mass spectrum such as mass spectrum 150, mass spectrum 151, mass spectrum 152, mass spectrum 153, or mass spectrum 201. The mass spectrometry data 403 may, for example, be first mass spectrometry data 114 or second mass spectrometry data 116.

The system 400 may be implemented on a computer system such as computer system 1000.

The system 400 comprises a mass spectrometry data input module 417, a candidate molecular sequence input module 419, a machine learning system 421, and an identification module 423.

The mass spectrometry data input module 417 is configured to obtain (or provide) the mass spectrometry data 403. The mass spectrometry data 403 may be, for example, received from memory 1006 of the computer 1002 that the system 400 is implemented (or performed) on. The mass spectrometry data 403 may be, for example, received from a storage medium 1004 of the computer 1002 that the system 400 (or the mass spectrometry data input module 417) is implemented (or performed) on. The mass spectrometry data 403 may be, for example, obtained (or received) from a user interface such as user input interface 1014 or a device such as device 1022. The mass spectrometry data 403 may be, for example, obtained (or received) via a network interface 1016.

The candidate molecular sequence input module 419 is configured to obtain (or provide) a set of candidate molecular sequences 405 for the mass spectrometry data 403, wherein each candidate molecular sequence corresponds to a respective candidate analyte. The set of candidate molecular sequences 405 may be, for example, received from memory 1006 of the computer 1002 that the system 400 (or the candidate molecular sequence input module 419) is implemented (or performed) on. The set of candidate molecular sequences 405 may be, for example, received from a storage medium 1004 of the computer 1002 that the system 400 (or the candidate molecular sequence input module 419) is implemented (or performed) on. The set of candidate molecular sequences 405 may be, for example, obtained (or received) from a user interface such as user input interface 1014 or a device such as device 1022. The set of candidate molecular sequences 405 may be, for example, obtained (or received) via a network interface 1016.

The set of candidate molecular sequences 405 may be generated by the system 400 (or the candidate molecular sequence input module 419).

The set of candidate molecular sequences 405 may be represented in the same ways as described above for molecular sequences 305.

The machine learning system 421 is trained to (or configured to or arranged to) identify one or more analytes represented in the mass spectrometry data 403. The machine learning system 421 comprises a first machine learning model 427, a second machine learning model 425, and a coefficient generating module 429.

The machine learning system 421 may have been trained via method 350. The machine learning system 421 may comprise the first neural network 309 and the second neural network 311. That is, the first machine learning model 427 may comprise (or be) the first neural network 309 and the second machine learning model 425 may comprise (or be) the second neural network 311.

The first machine learning model 427 is configured to transform (or encode) the mass spectrometry data 403 into a mass spectrometry data vector 407 in a latent (or vector) space 409. For example, the transformation of the mass spectrometry data 403 into a mass spectrometry data vector 407 may be performed by the first neural network 309. The transformation of the mass spectrometry data 402 into the mass spectrometry data vector 407 may comprise projecting the mass spectrometry data 402 into the latent space 409.

The second machine learning model 425 is configured to transform (or encode) each candidate molecular sequence (of the set of candidate molecular sequences 405) into a respective candidate molecular sequence vector 411 in the latent (or vector) space 409. For example, the transformation of the set of candidate molecular sequences 405 into candidate molecular sequence vectors 411 may be performed by the second neural network 311. The transformation of each candidate molecular sequence into the respective candidate molecular sequence vector 411 may comprise projecting that candidate molecular sequence into the latent (or vector) space 409.

The coefficient generating module 429 is configured to determine a set of analyte coefficients 413 for the set of candidate molecular sequences 405, each analyte coefficient in the set of analyte coefficients 413 being based on the mass spectrometry data vector 407 and the respective candidate molecular sequence vectors 411. In this way, each analyte coefficient 413 may correspond to a respective one of the candidate molecular sequences 405.

The number of analyte coefficients in the set of analyte coefficients 413 may be equal to the number of candidate molecular sequences in the set of candidate molecular sequences 405, in which case each candidate molecular sequence in the set of candidate molecular sequences 405 will have a respective analyte coefficient 413.

The set of analyte coefficients may be, for example, a vector. Each respective analyte coefficient may be, for example, a numerical value. For example, each respective analyte coefficient may be, for example, a natural number or a real number. Each analyte coefficient may be, for example, represented as a decimal value between 0 and 1. The total sum of the analyte coefficients may be 1. In this way, the decimal values may be configured to directly indicate (or identify) which analytes are represented in the mass spectrometry data (via whether a coefficient is zero or non-zero) and, where represented, what their relative abundances are (via the magnitude of a non-zero coefficient), the coefficients can be extracted directly from training data used to generate the training dataset. The closer to one an analyte coefficient is, the more abundant the respective analyte is. The ratio of the analyte coefficients may be the same as the ratio of abundances of the respective analytes.

Each analyte coefficient of the set of analyte coefficients 413 may determined by (or calculated from or as) the dot (or scalar) product of the mass spectrometry data vector 407 and the respective candidate molecular sequence vector 411.

The machine learning system 421 may be configured to output the set of analyte coefficients 413.

The identification module 423 may be configured to input the set of analyte coefficients 413 output by the machine learning system 421. The identification module 423 is configured to provide, for one or more of the candidate molecular sequences 405, a respective indication 415 of a match in the mass spectrometry data, based at least in part on the set of analyte coefficients 413.

Each indication 415 may be the same type of indication. Alternatively, the respective indications 415 for one or more of the candidate molecular sequences may differ from the respective indication 415 of other candidate molecular sequences in the set 405.

For example, one example type of indication is a binary 'yes'/'no' indication, wherein a binary 'yes' indication for a respective candidate molecular sequence indicates that the respective candidate molecular sequence is of (or corresponds to) an analyte present in the mass spectrometry data 403; and wherein a binary 'no' indication for a respective candidate molecular sequence indicates that the respective candidate molecular sequence is of (or corresponds to) an analyte not present in the mass spectrometry data 403.

Another example type of indication is a probability indication, wherein the probability indication for a respective candidate molecular sequence indicates a probability that the respective candidate molecular sequence is of (or corresponds to) an analyte present in the mass spectrometry data 403.

Another example type of indication is an identification of (e.g. the molecular sequence of or an analyte name corresponding to) the candidate molecular sequences that have a match in the mass spectrometry data 403. For example, this may be an identification of the candidate molecular sequences which have a probability of being (or corresponding to) an analyte present in the mass spectrometry data 403 which exceeds a threshold, wherein the threshold may be, for example, 50%, 60%, 70%, 80%, 90%, 95%, 99%, 99.5% or 99.9%.

It will be appreciated that further example types of indication may comprise combinations of the example types of indication listed above. It will also be appreciated that the identification module 423 may, instead of being separate from machine learning system 421, be part of machine learning system 421.

The system 400 may be configured to output the indication(s) 415 of a match, for example, via user output interface 1012. The indication(s) 415 may be, for example, displayed on a display screen to the user. It will be appreciated that the indication(s) 415 may be used in further processing. For example, the indication(s) 415 may be input to an alert system configured to trigger an alert in response to a respective indication 415 of a positive match in the mass spectrometry data 403 (e.g. a probability of that candidate molecular sequence being present in the mass spectrometry data 403 exceeding a threshold) for one or more pre-specified candidate molecular sequences.

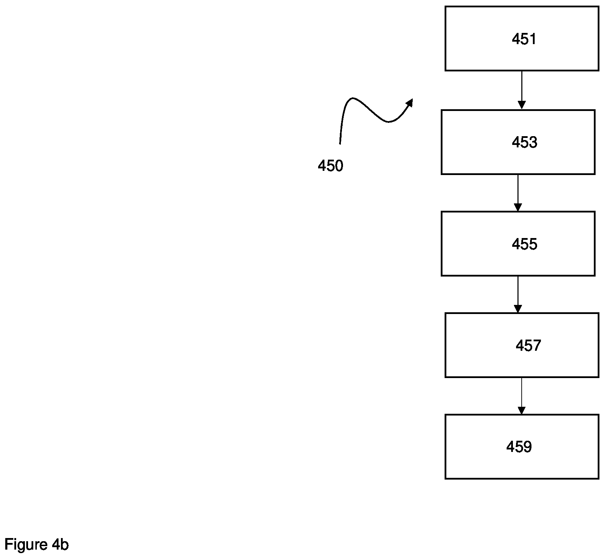

**Figure 4b** schematically illustrates an example method 450 of identifying one or more analytes represented in (chimeric) mass spectrometry data. The method 450 may be implemented on (or with or using) system 400.

The (chimeric) mass spectrometry data may, for example, be represented by a mass spectrum such as mass spectrum 150 or mass spectrum 201. The (chimeric) mass spectrometry data may, for example, be first mass spectrometry data 114 or second mass spectrometry data 116.

The mass spectrometry data may have been input to (or obtained by) mass spectrometry data input module 417.

The method 450 may be implemented on a computer system such as computer system 1000.

In method 450, a first step 451 comprises obtaining a set of candidate molecular sequences for the mass spectrometry data, wherein each candidate molecular sequence corresponds to a respective candidate analyte. The set of candidate molecular sequences may be, for example, received from memory 1006 of the computer 1002 that the method 450 is implemented (or performed) on. The set of candidate molecular sequences may be, for example, received from a storage medium 1004 of the computer 1002 that the method 450 is implemented (or performed) on. The set of candidate molecular sequences may be, for example, obtained (or received) from a user interface such as user input interface 1014 or a device such as device 1022. The set of candidate molecular sequences may be, for example, obtained (or received) via a network interface 1016. The set of candidate molecular sequences may be obtained by the candidate molecular sequence input module 419.

The set of candidate molecular sequences may be represented in the same ways as described above for molecular sequences 305.

In method 450, a second step 453 comprises transforming (or encoding) the mass spectrometry data into a mass spectrometry data vector in a latent (or vector) space.

Transforming the mass spectrometry data may be carried out using a first machine learning model, such as first machine learning model 427. Transforming the mass spectrometry data may be carried out using a first trained neural network, such as first neural network 309. That is, the mass spectrometry data may be input into a first trained neural network (or first machine learning model) which is configured to (or trained to) output a mass spectrometry data vector in a latent (or vector) space.

In method 450, a third step 455 comprises transforming (or encoding) each candidate molecular sequence into a respective candidate molecular sequence vector in the latent (or vector) space.

Transforming the set of candidate molecular sequences may be carried out using a second machine learning model, such as second machine learning model 425. Transforming the set of candidate molecular sequences may be carried out using a second trained neural network, such as second neural network 311. That is, one or more of the candidate molecular sequences may be input into a second trained neural network (or second machine learning model) which is configured to (or trained to) output a respective candidate molecular sequence vector in a latent (or vector) space. It will be appreciated that the first and second machine learning models may be implemented on the same machine learning model architecture as different operations.

It will be appreciated that all of the candidate molecular sequences may be input into the same second trained neural network (or same second machine learning model) or alternatively, that there may be multiple trained neural networks (or multiple other machine learning models) which are each configured to (or trained to) output a respective candidate molecular sequence vector in a latent (or vector) space, of which the second trained neural network (or second machine learning model) is one. In such a case, transforming the set of candidate molecular sequences may be carried out using a second trained neural network and further trained neural networks (or analogously a second machine learning model and further machine learning models, of type other than a neural network), wherein each trained neural network (or other machine learning model) may be arranged to input one or more of the candidate molecular sequences and output a respective candidate molecular sequence vector in a latent (or vector) space. The latent space where each respective candidate molecular sequence vector is output into may be the same latent space.

The first trained neural network (or first machine learning model) and the second trained neural network (or second machine learning model) may be jointly trained using a common loss function to output vectors in a common latent space. The common loss function may be common loss function 313 as described above. The common loss function may be formulated as a distance between discrete distributions representing predicted (or calculated) coefficients and true coefficients of chimeric mass spectrometry data. The common loss function may comprise a projection of a mass spectrometry data vector generated by the first neural network (or first machine learning model) on a corresponding molecular sequence vector generated by the second neural network (or second machine learning model). Alternatively, the common loss function may comprise a projection of a molecular sequence vector generated by the second neural network (or second machine learning model) on a corresponding mass spectrometry data vector generated by the first neural network (or first machine learning model). The projection may provide the predicted (or calculated) coefficients of the chimeric mass spectrometry data. The projection may directly provide the predicted (or calculated) coefficients of the chimeric mass spectrometry data The true coefficients of the chimeric mass spectrometry data may be input along with the chimeric mass spectrometry data during training and may be generated (or produced) as described in connection with generation of the training set above.

The first trained neural network may be a transformer and the second trained neural network may be a transformer. The first trained neural network may comprise a transformer layer (e.g. the first trained neural network may be an encoder as described above) and the second trained neural network may comprise a transformer layer e.g. the second trained neural network may be an encoder as described above).

One or more layer of the first trained neural network and/or one or more layer of the second trained neural network may use L2-norm regularization for layer weights. In particular, weight decay (e.g. with L2-norm regularization) may be used in transformer layers of the first and/or second neural networks. This use of weight decay may improve neural network convergence.

In method 450, a fourth step 457 comprises determining a set of analyte coefficients for the set of candidate molecular sequences, each analyte coefficient in the set of analyte coefficients being based on the mass spectrometry data vector and the respective candidate molecular sequence vector. Each analyte coefficient may optionally be determined based at least in part on a respective projection (or inner or dot product) of the mass spectrometry data vector and the respective candidate molecular sequence vector. The determined set of analyte coefficients may be represented in the same ways as set of analyte coefficients 307 described above. Determining the set of analyte coefficients for the set of candidate molecular sequences, each analyte coefficient in the set of analyte coefficients being based (directly) on the mass spectrometry data vector and the respective candidate molecular sequence vector, may be performed by coefficient generating module 429.

In method 450, a fifth step 459 comprises providing, for one or more of the candidate molecular sequences, a respective indication of a match in the mass spectrometry data, based at least in part on the set of analyte coefficients. The respective indications may be indications 415 as described above and may be used in further processing as described above. The providing, for one or more of the candidate molecular sequences, a respective indication of a match in the mass spectrometry data, based at least in part on the set of analyte coefficients may be performed by the identification module 423.

It will be appreciated that in some examples, the determined set of analyte coefficients may be a vector. Each element in the vector may correspond to a respective candidate molecular sequence for the mass spectrometry data The vector may be a vector of zero values and non-zero values. Each non-zero value may represent a candidate molecular sequence that is present in the mass spectrometry data and the relative abundance of that molecular sequence in the mass spectrometry data. Each zero value may represent a candidate molecular sequence that is not present in the mass spectrometry data. A higher (or greater or larger) non-zero value may represent a higher (or greater or larger) relative abundance. In this way, not only can the analytes present in the mass spectrometry data be determined using method 450 (from the set of analyte coefficients) but also their relative abundance. Moreover, the skilled person would appreciate that there are many other ways of determining relative abundance using method 450 - for example, determining a scaling of mass spectrometry data corresponding to the identified matched analytes which results in the mass spectrometry data under assessment and, from the scaling, determining a relative abundance of each identified matched analyte.

In this way, method 450 essentially formulates deconvolution of chimeric spectra for molecular sequence identification as a machine learning problem. In this problem, a) 'scoring' of the individual molecular sequences as matches for the mass spectrometry data; and b) 'deconvolution' of the chimeric spectra into spectra of individual molecular sequences are combined using a single loss function. Thus, method 450 provides an end-to-end scoring and deconvolution method for chimeric spectra that does not rely on subjective/heuristic feature engineering. In other words, method 450 can be used to generate a set of analyte matches (and their relative contributions) to a given item of chimeric mass spectrometry data, with machine learning techniques being used to identify the chimericity of the mass spectrometry data directly from the mass spectrometry data.

It will be appreciated that there may be provided a system arranged to carry out method 450. It will also be appreciated that there may be provided computer program which, when executed by one or more processors, causes the one or more processors to carry out method 450. The one or more processors may comprise processor 1008. It will also be appreciated that there may be provided a computer-readable medium storing such a computer program.

## Modifications

It will be appreciated that the methods described have been shown as individual steps carried out in a specific order. However, the skilled person will appreciate that these steps may be combined or carried out in a different order whilst still achieving the desired result.

It will be appreciated that embodiments of the invention may be implemented using a variety of different information processing systems. In particular, although the figures and the discussion thereof provide an exemplary computing system and methods, these are presented merely to provide a useful reference in discussing various aspects of the invention. Embodiments of the invention may be carried out on any suitable data processing device, such as a personal computer, laptop, personal digital assistant, mobile telephone, set top box, television, server computer, cloud etc. Of course, the description of the systems and methods has been simplified for purposes of discussion, and they are just one of many different types of system and method that may be used for embodiments of the invention. It will be appreciated that the boundaries between logic blocks are merely illustrative and that alternative embodiments may merge logic blocks or elements, or may impose an alternate decomposition of functionality upon various logic blocks or elements.

It will be appreciated that the above-mentioned functionality may be implemented as one or more corresponding modules as hardware and/or software. For example, the above-mentioned functionality may be implemented as one or more software components for execution by a processor of the system. Alternatively, the above-mentioned functionality may be implemented as hardware, such as on one or more field-programmable-gate-arrays (FPGAs), and/or one or more application-specific-integrated-circuits (ASICs), and/or one or more digital-signal-processors (DSPs), and/or one or more graphical processing units (GPUs), and/or other hardware arrangements. Method steps implemented in flowcharts contained herein, or as described above, may each be implemented by corresponding respective modules; multiple method steps implemented in flowcharts contained herein, or as described above, may be implemented together by a single module.

It will be appreciated that, insofar as embodiments of the invention are implemented by a computer program, then one or more storage media and/or one or more transmission media storing or carrying the computer program form aspects of the invention. The computer program may have one or more program instructions, or program code, which, when executed by one or more processors (or one or more computers), carries out an embodiment of the invention. The term "program" as used herein, may be a sequence of instructions designed for execution on a computer system, and may include a subroutine, a function, a procedure, a module, an object method, an object implementation, an executable application, an applet, a servlet, source code, object code, byte code, a shared library, a dynamic linked library, and/or other sequences of instructions designed for execution on a computer system. The storage medium may be a magnetic disc (such as a hard drive or a floppy disc), an optical disc (such as a CD-ROM, a DVD-ROM or a BluRay disc), or a memory (such as a ROM, a RAM, EEPROM, EPROM, Flash memory or a portable/removable memory device), etc. The transmission medium may be a communications signal, a data broadcast, a communications link between two or more computers, etc.

## Claims

1. A method of identifying one or more analytes represented in mass spectrometry data, the method comprising:

obtaining a set of candidate molecular sequences for the mass spectrometry data, wherein each candidate molecular sequence corresponds to a respective candidate analyte;
transforming the mass spectrometry data into a mass spectrometry data vector in a latent space;
transforming each candidate molecular sequence into a respective candidate molecular sequence vector in the latent space;
determining a set of analyte coefficients for the set of candidate molecular sequences, based on the mass spectrometry data vector and the candidate molecular sequence vectors;
providing, for one or more of the candidate molecular sequences, a respective indication of a match in the mass spectrometry data, based at least in part on the set of analyte coefficients.

2. The method of claim 1, wherein each analyte coefficient is determined based at least in part on a respective projection of the mass spectrometry data vector and the respective candidate molecular sequence vector.

3. The method of claim 1 or 2, wherein transforming the mass spectrometry data is carried out using a first trained neural network, and wherein transforming the set of candidate molecular sequences is carried out using a second trained neural network.

4. The method of claim 3, wherein the first trained neural network and the second trained neural network are jointly trained using a common loss function to output vectors in the latent space.

5. The method of claim 4, wherein the common loss function comprises one of:

a) a projection of a mass spectrometry data vector generated by the first neural network on a corresponding molecular sequence vector generated by the second neural network; and
b) a projection of a molecular sequence vector generated by the second neural network on a corresponding mass spectrometry data vector generated by the first neural network.

6. The method of any of claims 3-5, wherein the first trained neural network is a transformer and wherein the second trained neural network is a transformer.

7. The method of any of claims 3-6, where in the one or more analytes comprise one or more peptides.

8. A method of training a machine learning system to identify one or more analytes represented in mass spectrometry data, the method comprising:

providing a training set comprising a plurality of items of chimeric mass spectrometry data and, for each item of mass spectrometry data, a respective set of molecular sequences of analytes present in the mass spectrometry data and a respective set of analyte coefficients;
jointly training a first neural network and a second neural network using the training set;
wherein the first neural network takes as input mass spectrometry data and provides as output a mass spectrometry data vector, and the second neural network takes as input a molecular sequence and provides as output a molecular sequence vector,
wherein the step of jointly training optimizes the first and the second neural networks using a common loss function which comprises a similarity metric between a mass spectrometry data vector, a molecular sequence vector and the respective analyte coefficients.

9. The method of claim 8, wherein the first neural network and the second neural network share a common latent space.

10. The method of claim 8 or 9, further comprising a step of augmenting the training set prior to jointly training the first neural network and the second neural network.

11. The method of claim 10, wherein the augmenting comprises:

generating one or more additional molecular sequences that are similar to one of the molecular sequences of analytes present in the mass spectrometry data; and
including the one or more additional molecular sequences in the training set as example molecular sequences of analytes not present in the mass spectrometry data.

12. The method of claim 11, where in the one or more analytes comprise one or more peptides and the generating each additional molecular sequence comprises:
generating a first sequence that is identical to the one of the molecular sequences of peptides present in the mass spectrometry data and then reversing the generated first sequence except for the last amino acid.

13. The method of claim 11, wherein the one or more analytes comprise one or more peptides and the generating each additional molecular sequence comprises:
generating a second sequence that is identical to the one of the molecular sequences of peptides present in the mass spectrometry data except for one amino acid in the generated second sequence which is different from the one of the peptide sequences.

14. The method of any one of claims 10-13, wherein the augmenting comprises:

   generating one or more modified molecular sequences, wherein generating each modified molecular sequence comprises generating a third sequence that is identical to the one of the molecular sequences of analytes present in the mass spectrometry data and then permuting the sequence order of the generated third sequence; and including each of the one or more modified molecular sequences in the training set as an example of a molecular sequence of an analyte present in the mass spectrometry data.

15. The method of any preceding claim, wherein the chimeric mass spectrometry data represents at least three different peptides.

16. A system arranged to carry out a method according to any one of the preceding claims.

17. A computer program which, when executed by one or more processors, causes the one or more processors to carry out a method according to any one of claims 1-15.

18. A computer-readable medium storing a computer program according to claim 17.

Figure 1a

Figure 1b

Figure 1c

Figure 2

Figure 3a

351

350

353

Figure 3b

Figure 4a  400

```
                                      ┌──────────────┐
                                      │              │
                                      │     451      │
                                      │              │
                                      └──────┬───────┘
                                             │
                                             ▼
                                      ┌──────────────┐
              ╭───╮                   │              │
              │   ╰─→                 │     453      │
                                      │              │
        450                           └──────┬───────┘
                                             │
                                             ▼
                                      ┌──────────────┐
                                      │              │
                                      │     455      │
                                      │              │
                                      └──────┬───────┘
                                             │
                                             ▼
                                      ┌──────────────┐
                                      │              │
                                      │     457      │
                                      │              │
                                      └──────┬───────┘
                                             │
                                             ▼
                                      ┌──────────────┐
                                      │              │
                                      │     459      │
                                      │              │
                                      └──────────────┘
```

Figure 4b

| | Europäisches Patentamt<br>European Patent Office<br>Office européen des brevets | **EUROPEAN SEARCH REPORT** | **Application Number**<br>EP 24 19 7377 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TARIQ MUHAMMAD USMAN ET AL: "SpeCollate: Deep cross-modal similarity network for mass spectrometry data based peptide deductions",<br>PLOS ONE,<br>vol. 16, no. 10,<br>29 October 2021 (2021-10-29), page e0259349, XP093247992,<br>US<br>ISSN: 1932-6203, DOI: 10.1371/journal.pone.0259349<br>* the whole document *<br>-----  | 1-18 | INV.<br>G16B40/10<br>G16B40/20 |
| X | Litsa Eleni ET AL: "Spec2Mol: An end-to-end deep learning framework for translating MS/MS Spectra to de-novo molecules",<br>,<br>12 September 2021 (2021-09-12), XP093248558,<br>DOI: 10.26434/chemrxiv-2021-6rdh6<br>Retrieved from the Internet:<br>URL:https://chemrxiv.org/engage/api-gateway/chemrxiv/assets/orp/resource/item/613e83a7656369203b2a249b/original/spec2mol-an-end-to-end-deep-learning-framework-for-translating-ms-ms-spectra-to-de-novo-molecules.pdf<br>* the whole document *<br>-----<br>-/-- | 1-18 | |
| | | | **TECHNICAL FIELDS SEARCHED (IPC)**<br>G16B |

The present search report has been drawn up for all claims

| Place of search | Date of completion of the search | Examiner |
|---|---|---|
| Munich | 10 February 2025 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 19 7377

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GESSULAT SIEGFRIED ET AL: "Prosit: proteome-wide prediction of peptide tandem mass spectra by deep learning", NATURE METHODS, vol. 16, no. 6, 27 May 2019 (2019-05-27), pages 509-518, XP093194000, New York ISSN: 1548-7091, DOI: 10.1038/s41592-019-0426-7 Retrieved from the Internet: URL:http://www.nature.com/articles/s41592-019-0426-7> * the whole document * ----- | 1-18 | |

TECHNICAL FIELDS
SEARCHED      (IPC)

The present search report has been drawn up for all claims

| Place of search | Date of completion of the search | Examiner |
|---|---|---|
| Munich | 10 February 2025 | Díaz de Lezana, C |

EPO FORM 1503 03.82 (P04C01)

## REFERENCES CITED IN THE DESCRIPTION

### Patent documents cited in the description

- EP 4102509 A1

### Non-patent literature cited in the description

- **SINITCYN, P. et al.** Computational Methods for Understanding Mass Spectrometry-Based Shotgun Proteomics Data. *Annu. Rev. Biomed. Data Sci.*, 2018, vol. 1 (1), 207-234, https://www.annualreviews.org/doi/10.1146/annurev-biodatasci-080917-013516
- **DORFER V. et al.** CharmeRT: Boosting Peptide Identifications by Chimeric Spectra Identification and Retention Time Prediction. *J. Proteome Res.*, 2018, vol. 17 (8), 2581-2589
- **J PARKINSON et al.** Expressed sequence tags: an overview. *Methods Mol Biol.*, 2009, vol. 533, 1-12, https://link.springer.com/protocol/10.1007/978-1-60327-136-3 1
- **S GESSULAT et al.** Prosit: proteome-wide prediction of peptide tandem mass spectra by deep learning. *Nat Methods.*, 27 May 2019, vol. 16 (6), 509-518, https://pubmed.ncbi.nlm.nih.gov/31133760/
- **S HELLER**. InChl, the IUPAC International Chemical Identifier. *Journal of Cheminformatics*, 2015, vol. 7, https://jcheminf.biomedcentral.com/articles/10.1186/s13321-015-0068-4
- **WIGH DS** ; **GOODMAN JM** ; **LAPKIN AA**. A review of molecular representation in the age of machine learning. *WIREs Comput Mol Sci.*, 2022, https://doi.org/10.1002/wcms.1603
- **VASWANI, A. et al.** Attention Is All You Need. *arXiv*, 2017, https://arxiv.org/abs/1706.03762
- **CHUNG, J. et al.** Empirical Evaluation of Gated Recurrent Neural Networks on Sequence Modeling. *arXiv*, 2014, https://arxiv.org/abs/1412.3555
- **YILMAZ, M. et al.** De novo mass spectrometry peptide sequencing with a transformer model. *bioRxiv*, 2022, https://doi.org/10.1101/2022.02.07.479481
- **ZHANG G et al.** Three Mechanisms of Weight Decay Regularization. *arXiv*, 2018, https://doi.org/10.48550/arXiv.1810.12281
- **ZHOU, X-X. et al.** pDeep: Predicting MS/MS Spectra of Peptides with Deep Learning. *Anal. Chem.*, 2017, vol. 89 (23), 12690-12697, https://doi.org/10.1021/acs.analchem.7b02566

- **T GESSULAT, S. et al.** Prosit: proteome-wide prediction of peptide tandem mass spectra by deep learning. *Nat. Methods*, 2019, vol. 16, 509-518, https://pubmed.ncbi.nlm.nih.gov/31133760
- **J B. MULLER**. The proteome landscape of the kingdoms of life. *Nature*, 2020, vol. 582, 592-596, https://doi.org/10.1038/s41586-020-2402-x
- **P E. GEYER et al.** Plasma Proteome Profiling to detect and avoid sample-related biases in biomarker studies. *EMBO Mol Med*, 2019, https://doi.org/10.15252/emmm.201910427
- **Y BIAN et al.** Robust, reproducible and quantitative analysis of thousands of proteomes by micro-flow LC-MS/MS. *Nat Commun*, 2020, vol. 11, 157, https://doi.org/10.1038/s41467-019-13973-x
- **D B. BEKKER-JENSEN et al.** *An Optimized Shotgun Strategy for the Rapid Generation of Comprehensive Human Proteomes*, 2017, https://doi.org/10.1016/j.cels.2017.05.009
- **C D. KELSTRUP et al.** Performance Evaluation of the Q Exactive HF-X for Shotgun Proteomics. *J. Proteome Res.*, 2018, vol. 17 (1), 727-738, https://doi.org/10.1021/acs.jproteome.7b00602
- **K SHARMA**. *Ultradeep Human Phosphoproteome Reveals a Distinct Regulatory Nature of Tyr and Ser/Thr-Based Signaling*, 2014, https://doi.org/10.1016/j.celrep.2014.07.036
- **K KRUG et al.** *Proteogenomic Landscape of Breast Cancer Tumorigenesis and Targeted Therapy*, 2020, https://doi.org/10.1016/j.cell.2020.10.036
- **A STUKALOV et al.** Multilevel proteomics reveals host perturbations by SARS-CoV-2 and SARS-CoV. *Nature*, 2021, vol. 594, 246-252
- **WANG, D. et al.** A deep proteome and transcriptome abundance atlas of 29 healthy human tissues. *Mol. Syst. Biol.*, 2019, vol. 15 (2), e8503, https://doi.org/10.15252/msb.20188503
- **J ELIAS et al.** Target-Decoy Search Strategy for Mass Spectrometry-Based Proteomics. *Methods Mol Biol.*, 2010, vol. 604, 55-71, https://link.springer.com/protocol/10.1007/978-1-60761-444-9 5

- **J ELIAS et al.** Comparative evaluation of mass spectrometry platforms used in large-scale proteomics investigations. *Nat Methods*, 2005, vol. 2, 667-675, https://doi.org/10.1038/nmeth785

- **X WANG**. Target-Decoy-Based False Discovery Rate Estimation for Large-Scale Metabolite Identification. *J Proteome Res.*, 2018, https://doi.org/10.1021/acs.jproteome.8b00019